(19) Europäisches Patentamt
European Patent Office
Office européen des brevets

(11) EP 4 609 869 A1

(12) EUROPEAN PATENT APPLICATION

(43) Date of publication:
03.09.2025 Bulletin 2025/36

(51) International Patent Classification (IPC):
A61K 31/737 (2006.01)     A61K 47/36 (2006.01)
A61P 17/00 (2006.01)      A61P 31/00 (2006.01)

(21) Application number: 24160979.1

(22) Date of filing: 01.03.2024

(52) Cooperative Patent Classification (CPC):
(C-Sets available)
A61K 31/737; A61K 47/36; A61L 15/225;
A61L 15/40; A61L 15/60; A61P 17/00; A61P 31/00;
C02F 1/286                              (Cont.)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(71) Applicant: Freie Universität Berlin
14195 Berlin (DE)

(72) Inventors:
• CHANDNA, Sanjam
  10717 Berlin (DE)
• BLOCK, Stephan
  14482 Potsdam (DE)
• BHATIA, Sumati
  14197 Berlin (DE)
• HAAG, Rainer
  12209 Berlin (DE)

(74) Representative: Maikowski & Ninnemann
Patentanwälte Partnerschaft mbB
Postfach 15 09 20
10671 Berlin (DE)

(54) **SULFATED LIGNIN FOR USE IN PATHOGEN ABSORPTION, IN WATER PURIFICATION, AS HYDROGEL, AS MEDICAMENT, AS DRUG CARRIER AND FOR MUCUS REPLACEMENT**

(57) The present invention relates to novel uses of sulfated lignin, such as its use as compound for adsorbing at least one of a microorganism, a pathogen, pollen, and spores. The invention further relates to a method for preparing a hydrogel comprising sulfated lignin.

FIG 4A

EP 4 609 869 A1

(52) Cooperative Patent Classification (CPC): (Cont.)

C-Sets
**A61L 15/225, C08L 33/08;**
**A61L 15/225, C08L 97/005**

**Description**

The present invention relates to medical and non-medical uses of sulfated lignin according to claims 1, 6, 7, and 10, to an article comprising sulfated lignin according to claim 11, and to a method for preparing a hydrogel comprising sulfated lignin according to claim 15.

Lignin represents an almost endless raw material produced through photosynthesis, which is currently significantly underutilized.[11, 12] As the second most abundant organic compound on earth, it is one of the main components of biomass which is thrown away in large quantities (approximately 70-100 Mio tons/year) as a by-product of paper and pulp industries.[13, 14] It has an irregular 3D structure, which is mainly built from three subunits, *i.e.,* guaiacyl (G), p-hydroxyphenyl (H), and syringyl (S). [1, 15-22] The complex organic structure of lignin provides this polymer with valuable properties, such as antioxidant, adhesiveness, UV-barrier, and antimicrobial properties. Owing to its highly diverse structure, good biocompatibility, and abundance in nature, lignin has the potential for biological applications.[23] Lignin and lignin-based hydrogels have been explored for different applications in literature due to the fascinating chemistry of lignin, decorated with numerous polyphenolic groups.[21, 24-26]

Sulfated and sulfonated lignin as well as various methods for manufacturing sulfated and sulfonated lignin are known, e.g., from US 2,688,611 A, US 5,013,825 A, US 5,049,661 A, and US 5,043,434 A.

Hydrogels are three-dimensional networks of hydrophilic polymers that can absorb and retain large amounts of water or biological fluids while maintaining their structural integrity.[1, 2] Mucus hydrogels that line biointerfaces play a vital role in both shielding the body from invading pathogens and facilitating the proper functioning of the underlying cells.[3] Mucus forms the first line of defense for protecting epithelial cells by acting as a physical barrier for the passage of deleterious molecules.[4] It allows the entry of nutrients and other vital molecules, but protects our body from pathogens such as certain dangerous viruses and bacteria.[5] Most of the mucus is water (~95 % w/w), the rest being mucins, globular proteins, salts, lipids, DNA, and cellular debris.[6] The pore size of mucus hydrogels *in vivo* lies in the range of a few hundred nanometers, excluding larger objects such as viruses, bacteria and yeast cells.[6] Objects less than the pore size, for example, infectious agents, are caught in the mucin network by restricting to the ligands present on the tip of the glycans and are removed from the mucosa.[7] Hence, along with acting as a barrier by affinity sorting, the mucus gels play the role of a filter.[8] These properties inspired the development of mucin-mimicking macromolecules and mucus-mimicking hydrogels. [9, 10]

It is an object of the present invention to provide novel uses for sulfated lignin.

This object is achieved with the uses indicated in claim 1. These novel uses of sulfated lignin are the use of lignin as a compound for adsorbing a microorganism, a pathogen, pollen, and /or spores. In an embodiment, and for legal reasons only, these uses are limited to non-medical (in particular non-therapeutic and non-diagnostic) uses, i.e., to uses, wherein methods for treatment of the human or animal body by surgery or therapy as well as diagnostic methods practiced on the human or animal body are excluded. Expressed in other words, the sulfated lignin is used as antimicrobial, anti-pathogenic, anti-pollen, and/or anti-spore compound.

The term "microorganism" as used herein refers to commensal, mutualistic, or pathogenic microorganisms found in and on all multicellular organisms, including plants. Microorganisms include bacteria, archaea, protists, fungi, and viruses.

The term "pathogen" as used herein is to be understood as describing an infectious microorganism or agent, such as a virus, a bacterium, a protozoan, a prion, a viroid, or a fungus. Thus, there is a partially overlap between the term "microorganism" and the term "pathogen", wherein the term "microorganism" also comprises non-infectious microorganisms, wherein the term "pathogen" also comprises infectious agents that are not classified as microorganisms.

The presently claimed and described novel uses of lignin are characterized, amongst others, by the following positive properties:

- Renewable Resource Utilization: Lignin is a byproduct of the paper and biofuel industries, which is thrown away in large quantities (up to approximately 100 Mio tons/year). By repurposing lignin for industrial or agricultural applications, the disclosure adds value to this renewable resource, contributing to a circular economy.

- Reduced Dependency on Synthetic Chemicals: The lignin-based application forms offer an eco-friendly alternative to traditional synthetic pesticides and coatings. In addition, lignin offers (a) high efficiency in microbial inactivation, (b) environmental sustainability, (c) limited toxicity, and (d) low cost and scalability.

- Enhanced Soil Health: As a natural component of plant cell walls, lignin has the potential to enrich the soil with organic matter, promoting soil health and fertility over time. Another advantage of the presently described and claimed lignin-based application forms is their UV protection ability, due to the inherent UV protection ability of lignin.

- Economic Opportunities for Farmers: The adoption of the presently described and claimed lignin-based application forms will result in decreased production costs for farmers, as well as potential economic opportunities in lignin production and processing.

- Mitigation of Environmental Impact: By reducing the use of synthetic chemicals in agriculture, the present disclosure contributes to mitigating the environmental impact associated with conventional farming practices, including soil and water contamination. The present disclosure aligns with the broader goals of sustainable agriculture and the development of bio-based solutions, fostering a more resilient and environmentally conscious agricultural sector.

Since lignin comes from nature and is highly abundant, the use of sulfated lignin as a watersoluble, biodegradable, self-adhesive antipathogenic agent for plants without contaminating the soil is a very attractive and a particularly appropriate application of sulfated lignin.

In an embodiment, the sulfated lignin is used as solid, in form of a hydrogel, or in form of a spray.

A spray of sulfated lignin can be conveniently produced since sulfated lignin is highly soluble in water and aqueous solutions. A spray can be easily applied to surfaces that are to be equipped with an antimicrobial or anti-pathogenic coating. An antimicrobial spray can also be used to treat plants, in particular the leaves and roots of plants. Such a spray can be very useful in preventing infections in plants. An antiviral spray is a particular appropriate example of an antimicrobial spray. An application of the antiviral spray against the tobacco mosaic virus (TMV) is a particularly appropriate application. An antibacterial spray is another particular appropriate example of an antimicrobial spray. An application of the antibacterial spray against *Erwinia amylovora* (being the causative bacterium of fire blight which is a great threat to apple and pear production worldwide) is a particularly appropriate application.

A hydrogel is another particularly appropriate application form of sulfated lignin. It is possible to modulate the chemical and physical properties of a hydrogel comprising sulfated lignin. In doing so, it is possible to mimic native mucus and to provide an artificial mucus that is able to combine filtering properties based on specific interactions with pathogens and microorganisms with an adjustable size selection. Such a combination is typically a hallmark of mucus. Hydrogel comprising sulfated lignin has superabsorbent properties and shows resistance to enzyme degradation, as tested with hyaluronidase. Hydrogels containing sulfated lignin showed antiviral activity against herpes simplex virus-1 (HSV-1), wherein the reduction in plaque forming units (PFU) was by 5 log units ($10^5$-fold inhibition). The sulfated lignin hydrogels also exhibited promising antibacterial activity against E. coli with a colony forming units (CFU) reduction by more than 4 log units (which is approximately $10^4$-fold inhibition).

A powder is a particularly appropriate application form of solid sulfated lignin.

Sulfated lignin may have various degrees of sulfation. The degree of sulfation is defined by the relative amount of hydroxyl groups (-OH) which are substituted by sulfate groups ($-O-SO_3$-Me). In this respect, "Me" denotes a metal atom, such as a monovalent metal atom. Alkali metal atoms such as lithium (Li), sodium (Na), and potassium (K) are particularly appropriate alkali metal atoms, wherein sodium and potassium, in particular sodium, are the most appropriate alkali metal atoms. Within the present disclosure, the non-dissociated form of sulfate groups (and other chemical groups) and the dissociated form of sulfate groups (and other chemical groups) are considered to be identical. Thus, the sulfate group can be represented in the forms $-O-SO_3$-Me and $-O-SO_3^-$ Me$^+$ that are to be considered identical from a chemical point of view. Thus, the beforementioned explanations with respect to (monovalent) metal atoms are also valid for (monovalent) metal ions.

In an embodiment, the sulfated lignin has a degree of sulfation lying in a range from 10 % to 100 %, in particular from 20 % to 95 %, in particular from 30 % to 90 %, in particular from 40 % to 85 %, in particular from 50 % to 80 %, in particular from 60 % to 70 %. It turned out that the degree of sulfation is decisive for the porosity of a hydrogel formed from the sulfated lignin. By adjusting the degree of sulfation, the porosity of a hydrogel formed from the sulfated lignin can be tuned over several orders of magnitude, ranging from pores in the nanometer scale to pores in the sub-millimeter scale.

While there are different methods to manufacture (or extract) lignin from plant material, the sulfated lignin is made, in an embodiment, from kraft lignin, i.e., from lignin produced by the so-called kraft process. Then, the sulfated lignin is sulfated kraft lignin. Kraft lignin does not comprise to a relevant extent sulfonate groups so that the sulfation of kraft lignin can be achieved easier than the sulfation of lignin obtained with different manufacturing processes that already introduce functional groups other than sulfate groups into the lignin.

In an embodiment, the sulfated lignin is used for water purification. Water purification is a particularly appropriate example for a novel use of lignin since the access to drinkable water also in regions of the earth that do not provide enough sufficiently pure groundwater is one of the key elements in providing a basis of existence for humans and animals.

In an embodiment, the sulfated lignin is used - besides using it as antimicrobial, anti-pathogenic, anti-pollen, and/or anti-spore agent - for storing water in soil. Thus, sulfated lignin can contribute to enhanced soil health not by only increasing the organic matter in soil but also by positively influencing the water balance of soil.

In an aspect, the present invention relates to the use of sulfated lignin for storing and sustained releasing water, i.e., as water storing element in a water retention system - independent on the ability of the sulfated lignin to adsorb microorganisms, pathogens, pollen and/or spores. There are many applications for such a water retention system enabling a sustained release of water. Typically, all applications that require a more or less constant water release over an extended period of time are appropriate applications of such a water retention system. In agriculture, such a water retention system can be used for hydrating plants (such as trees like street trees), seedlings, and/or seed bombs over an extended period of time in particular in regions in which large amounts of rain fall within short time, wherein there are longer dry periods between individual rainfalls. Then, the water binding and releasing capacity of sulfated lignin can be advantageously used for providing a more or less constant source of water for plants and other agricultural elements also during dry times.

In an embodiment, and for legal reasons only, the before-mentioned use is limited to a non-medical (in particular non-therapeutic and non-diagnostic) use, i.e., to a use, wherein methods for treatment of the human or animal body by surgery or therapy as well as diagnostic methods practiced on the human or animal body are excluded.

In an embodiment, the sulfate groups are not the only functionalization of the sulfated lignin. Rather, in this embodiment, the sulfated lignin also comprises a functionalization of some of its hydroxyl groups with at least one type of sugar residue, such as a sialic acid residue, a fucose residue, a galactose residue, or a mannose residue. Then, the sulfated lignin comprises different functionalizations (sulfate and sugar functionalization) that enable a particularly appropriate interaction with microorganisms and/or pathogens and increase the overall binding capacity of the sulfated lignin for such microorganisms and/or pathogens.

In an aspect, the present invention relates to a method for adsorbing at least one of a microorganism, a pathogen, a pollen, and spores by applying sulfated lignin onto to the surface of an article or a plant, by applying sulfated lignin into soil, and/or by equipping an article (such as a gas or water purification device) with sulfated lignin in an appropriate physical state and guiding a fluid (e.g., a gas like air or a liquid like water or an aqueous solution) across the sulfated lignin such that the sulfated lignin is able to interact and to adsorb at least one of a microorganism, a pathogen, a pollen, and spores present in the fluid.

In an aspect, the present invention relates to a method for storing and releasing water, wherein the method comprises the steps explained in the following. First, a water retention system is provided. The water retention system comprises a water retention element that comprises a partially or fully dehydrated hydrogel of sulfated lignin, i.e., a swellable polymer network of sulfated lignin that is able to absorb water or an aqueous solution. Afterwards, the water retention element is brought in contact with water or an aqueous solution. As a result, the water retention element will absorb this water or aqueous solution; the water retention element is hydrated. Afterwards, the water retention system with the hydrated water retention element is placed in an environment in which the water retention system shall release the stored water or aqueous solution. Such release is then allowed to take place over an extended period of time. Typically, a humidity gradient between the water retention system and the surrounding to which the water is to be released is decisive for the speed and amount of water released by the water retention system. In an embodiment, additional physical forces are applied to the water retention system to achieve the desired water release. This can be done, e.g., by applying pressure to the water retention element or the water retention system. The water is then "squeezed out" of the water retention element.

In an aspect, the present invention relates to the medical use of sulfated lignin, i.e., to sulfated lignin for use as a medicament. Various medical applications are possible for sulfated lignin, in particular medical applications involving an antimicrobial or anti-pathogenic effect of the sulfated lignin. In an embodiment, a medicament (drug) comprising sulfated lignin is used as antibacterial or antiviral drug, in particular as drug acting against bacteria such as E. coli or against viruses such as herpes viruses like HSV-1, influenza viruses such as influenza A viruses, or coronaviruses like SARS-CoV-2.

In an embodiment, the medicament is designed and configured to be applied to a patient's skin. Such external application of novel medicaments typically requires less stringent regulatory approval than systemically acting drugs for internal applications such as oral or transvenous administration. Since the patient's skin is a natural barrier against microorganisms and pathogens, a medicament comprising sulfated lignin can improve, temporarily replace, or assist the skin's barrier function. If the skin's barrier function is impaired, e.g., due to skin injury because of burning, a cream or and ointment comprising sulfated lignin can at least partially take over some of the skin's barrier functions and can act as anti-microbial and/or anti-pathogenic barrier reducing the risk of infections of the patient.

In an embodiment, the medicament is a mucus replacement. Mucus is a complex hydrogel acting as a defensive and protective barrier in various parts of the human body. Structure and composition of mucus play an important role in maintaining barrier properties while acting as a filter for the diffusion of biomolecules and pathogens. The rise in viral infections has underscored the importance of advancing research on mucus-mimicking hydrogels for the efficient design of antiviral agents. Since the gram-scale synthesis of biocompatible, lignin-based virus-binding inhibitors that reduce waste and ensure long-term availability is possible according to an aspect of the present invention, the sulfated lignin is particularly appropriate to be used as mucus replacement. The sulfated lignin is a potent binding partner for many viruses including SARS-CoV-2 and herpes viruses. Crosslinking the sulfated lignin yields hydrogels that mimic native mucus with respect to surface functionality and rheology. It was found that the degree of sulfation has a strong impact on the mesh size distribution of the hydrogels; this provides a new means to fine-tune steric and electrostatic contributions of the virus-hydrogel interaction. This feature strongly impacts the sequestration capability of the lignin-based hydrogel, which was demonstrated by infection inhibition assays involving human herpes simplex virus-1, influenza A viruses, and the bacterium *E. coli*. For HSV-1 and *E. coli,* these measurements showed a reduction in PFU in case of HSV-1 and in CFU in case of E. *coli* by more than four orders of magnitude, indicating potent inhibition by the lignin-based hydrogels. Taken together, the sulfated lignin hydrogel is an excellent scaffold for large-scale synthesis of sustainable, biocompatible, and highly efficient pathogen-binding inhibitors like mucus replacement.

In an aspect, the present invention relates to the use of sulfated lignin as a drug delivery compound. While such drug delivery compound does not necessarily exhibit a pharmaceutical activity on its own, it is a carrier or vehicle for pharmaceutically active substances, in particular drugs. Due to the antimicrobial and anti-pathogenic properties of sulfated lignin, a drug delivery compound comprising sulfated lignin also has antimicrobial and/or anti-pathogenic properties. This may increase the stability of a drug delivered by such drug delivery compound.

In an embodiment, the drug delivery compound comprising sulfated lignin has tunable drug releasing properties. This can be achieved by using different degrees of cross-linking of a hydrogel comprising sulfated lignin and being used as structural element of the drug delivery compound. The higher the amount of cross-linkings between individual units, the longer takes a degradation of a hydrogel comprising the sulfated lignin. This in turn leads to a prolonged time for releasing a drug encapsulated by such hydrogel comprising sulfated lignin.

In an aspect, the present invention relates to a medical method of treating a patient in need of such treatment with a drug comprising sulfated lignin. Such a method is typically an antimicrobial and/or anti-pathogenic therapeutic method.

In an aspect, the present invention relates to a method of delivering a drug to a patient in need of such drug with the help of a drug delivery compound, wherein the drug delivery compound comprises sulfated lignin. Typically, the drug delivery compound comprises a hydrogel synthesized from the sulfated lignin.

In an aspect, the present invention relates to an article comprising sulfated lignin. The article can be any item that is to be equipped with antimicrobial and/or anti-pathogenic properties and/or that shall have a high water-binding capacity.

In an embodiment, the article comprises an article core having a surface, wherein the sulfated lignin has been applied onto the surface in form of a coating, in particular an antimicrobial and/or an anti-pathogenic coating. Then, the surface of the article represents a highly effective barrier against microorganisms, pathogens, pollen, and/or spores. Transmission of microbes through exposure with contaminated surfaces is an indispensable pathway for the outspread of various infections. As revealed by the European Center for Disease Prevention and Control, more than 4 million people acquire a Healthcare-Associated Infection (HCAI) each year, which results in 37,000 deaths. Combatting HCAIs is a significant problem for the healthcare sector around the globe. It is not possible to always clean, disinfect or use strong chemicals on surfaces to prevent the growth of germs. Therefore, antimicrobial coatings have emerged as one of the most interesting technologies to prevent growth and to subsequently kill disease-causing microorganisms. The coating of a surface with an antimicrobial material leads to a more secure and long-term solution of this problem.

According to prior art, the approaches towards antimicrobial surface coatings either involve complex synthetic polymers or metal-based materials as additives (metal ions, such as silver and quaternary ammonium compounds). There are obvious challenges associated with these approaches when it comes to their environmental sustainability and large-scale applications. Since there is a need for safe and sustainable alternatives in the coating industry, various oils and waxes have been tested as material coatings. However, they often lack sufficient durability. Sulfated lignin represents an almost ideal material for favorably acceptable and affordable antimicrobial coatings since it meets the following requirements: (a) high efficiency in microbial inactivation, (b) environmental sustainability, (c) limited toxicity, and (d) low cost and scalability. Besides its antimicrobial and anti-pathogenic properties, the complex organic structure of sulfated lignin provides this polymer with additional valuable properties, such as antioxidant, adhesiveness, and UV-barrier properties.

In an embodiment, the article is a furniture item used in hospital or in a similar medical environment. Equipping the surface of such a furniture item with an antimicrobial coating comprising sulfated lignin is a particularly appropriate application of exploiting the antimicrobial/anti-pathogenic properties of sulfated lignin in an environment in which contaminations should be avoided but are typically difficult to control.

In an embodiment, the sulfated lignin forms part of a swellable polymer network that is configured to absorb water or an aqueous solution and to form a hydrogel upon absorbing water or an aqueous solution. The swellable polymer network (i.e., the dried hydrogel) typically has a very high swelling capacity and can thus absorb a multiple of its own weight of water or an aqueous solution, in particular a two-fold to 100-fold of its own weight, in particular a fivefold to 90-fold, in particular a tenfold to 80-fold, in particular a 20-fold to 70-fold, in particular a 30-fold to 60-fold, in particular a 40-fold to 50-fold of its own weight..

In an embodiment, the article is a medical, veterinary, or sanitary article. Any kind of medical dressing such as a wound dressing or a wound coverage is an appropriate example of a medical or veterinary article (which may be classified as medical product). A sanitary pad, a sanitary tampon, and a diaper are particularly appropriate examples of a sanitary article. The combination of high water absorbing capacity and antimicrobial/anti-pathogenic properties make hydrogels (or hydrogel precursors like swellable polymer networks) comprising sulfated lignin highly potent components of such medical, veterinary, or sanitary articles.

In an aspect, the present invention relates to a method for preparing a hydrogel comprising sulfated lignin. This method comprises the steps explained in the following.

In a first step, an aqueous solution of sulfated lignin is provided.

Afterwards, an aqueous solution of polyacrylic acid is added to the aqueous solution of sulfated lignin. This results in a reaction mixture.

The reaction mixture is stirred for first period of time to achieve a good mixture of the individual components present in the reaction mixture.

Afterwards, a free radical initiator is added to the reaction mixture. This free radical initiator initiates a radical polymerization. Due to this initiated radical polymerization, cross-linking and copolymerization between the sulfated lignin and the polyacrylic acid takes place by initial formation of ester linkages. Briefly, lignin acryl ester is formed, which is polymerized with acrylic acid to form a hydrogel. As a result, a hydrogel is obtained during a second period of time during which the polymerization reaction takes place.

Subsequently, it is possible (but not necessary) to purify the obtained hydrogel by one or more appropriate purification methods.

In an embodiment, the aqueous solution of sulfated lignin comprises the sulfated lignin in an amount of 0.1 % to 5 % weight per volume (w/v), in particular of 0.2 % to 4.5 % (w/v), in particular of 0.3 % to 4 % (w/v), in particular of 0.4 % to 3.5 % (w/v), in particular of 0.5 % to 3 % (w/v), in particular of 0.6 % to 2.5 % (w/v), in particular of 0.7 % to 2 % (w/v), in particular of 0.8 % to 1.5 % (w/v), in particular of 0.9 % to 1 % (w/v).

In an embodiment, the aqueous solution of polyacrylic acid has a concentration of polyacrylic acid lying in a range of from 0.5 % to 5 % (w/v), in particular from 1 % to 4.5 % (w/v), in particular from 1.5 % to 4 % (w/v), in particular from 2 % to 3.5 % (w/v), in particular from 2.5 % to 3 % (w/v).

In an embodiment, the step of stirring the reaction mixture is carried out at a temperature lying in a range from 5 °C to 50 °C, in particular from 10 °C to 45 °C, in particular from 15 °C to 40 °C, in particular from 20 °C to 35 °C, in particular from 25 °C to 30 °C, in particular at or around room temperature.

In an embodiment, the stirring in the stirring step is carried out at a speed from 50 rounds per minute (rpm) to 1000 rpm, in particular from 100 rpm to 900 rpm, in particular from 200 rpm to 800 rpm, in particular from 300 rpm to 700 rpm, in particular from 400 rpm to 600 rpm, in particular from 450 rpm to 500 rpm.

In an embodiment, the first period of time lies in a range from 30 seconds to 1 hour, in particular from 1 minute to 55 minutes, in particular from 2 minutes to 50 minutes, in particular from 3 minutes to 45 minutes, in particular from 4 minutes to 40 minutes, in particular from 5 minutes to 35 minutes, in particular from 6 minutes to 30 minutes, in particular from 7 minutes to 25 minutes, in particular from 8 minutes to 20 minutes, in particular from 9 minutes to 15 minutes, in particular from 10 minutes to 12 minutes.

In an embodiment, the free radical initiator is at least one of an inorganic peroxide such as ammonium persulfate (APS), sodium persulfate, and potassium persulfate; an organic peroxide such as di-*tert*-butyl peroxide, benzoyl peroxide, methyl ethyl ketone peroxide, and acetone peroxide; and an azo compound such as azobisisobutyronitrile and 1,1'-azobis(cyclohexanecarbonitrile).

In an embodiment, the free radical initiator is used in a concentration lying in a range of from 0.5 % to 5 % (w/v), in particular from 1 % to 4.5 % (w/v), in particular from 1.5 % to 4 % (w/v), in particular from 2 % to 3.5 % (w/v), in particular from 2.5 % to 3 % (w/v).

In an embodiment, cross-linking and copolymerization is allowed to take place at a temperature lying in a range from 20 °C to 95 °C, in particular from 30 °C to 90 °C, in particular from 40 °C to 80 °C, in particular from 50 °C to 75 °C, in particular from 60 °C to 70 °C.

In an embodiment, the second period of time lies in a range from 30 seconds to 1 hour, in particular from 1 minute to 55 minutes, in particular from 2 minutes to 50 minutes, in particular from 3 minutes to 45 minutes, in particular from 4 minutes to 40 minutes, in particular from 5 minutes to 35 minutes, in particular from 6 minutes to 30 minutes, in particular from 7 minutes to 25 minutes, in particular from 8 minutes to 20 minutes, in particular from 9 minutes to 15 minutes, in particular from 10 minutes to 12 minutes.

In an embodiment, the optional purification step is carried out by dialyzing the obtained hydrogel against an appropriate buffer such as phosphate buffered saline (PBS). This facilitates the removal of unreacted free radical initiator and other contaminants present in the solution containing the developed hydrogel. A dialysis time lying in a range from 1 hour to 24 hours, in particular 2 hours to 20 hours, in particular 3 hours to 18 hours, in particular 4 hours to 15 hours, in particular 6 hours to 12 hours, in particular 8 hours to 10 hours is a particularly appropriate dialysis time.

In an embodiment, a dialysis membrane used for the dialysis has a molecular weight cut-off lying in a range from 250 Da to 5 kDa, in particular from 500 Da to 4 kDa, in particular from 750 Da to 3 kDa, in particular from 1 kDa to 2 kDa.

In an aspect, the present invention relates to a method for adjusting the pore size of pores being present in a hydrogel formed from sulfated lignin, in particular a hydrogel manufactured by a method according to the preceding explanations. This method for adjusting the pore size comprises an adjustment of the degree of sulfation of the sulfated lignin to adjust the pore size in the manufactured hydrogel. In this context, a higher degree of sulfation results in a smaller pore size.

**EP 4 609 869 A1**

In an embodiment, the degree of sulfation lies in a range from 70 % to 84.9 %, in particular from 75 % to 80 %, in particular from 77 % to 79 %, in particular around or at 78 %, wherein the pore size (measured in micrographs obtained by scanning electron microscopy (SEM)) of the resulting hydrogel lies in a range from 5 $\mu$m to 15 $\mu$m, in particular from 7 $\mu$m to 12 $\mu$m, in particular from 9 $\mu$m to 11 $\mu$m. In an embodiment, the degree of sulfation lies in a range of from 85 % to 95 %, in particular from 87 % to 93 %, in particular from 89 % to 91 %, in particular around or at 90 %, wherein the pore size of the resulting hydrogel (measured in micrographs obtained by SEM) lies in a range from 500 nm to 4.9 $\mu$m, in particular from 600 nm to 4 $\mu$m, in particular from 700 nm to 3.5 $\mu$m, in particular from 800 nm to 3 $\mu$m, in particular from 900 nm to 2 nm, in particular from 1 $\mu$m to 1.5 $\mu$m.

In an embodiment, the concentration of the sulfated lignin in the hydrogel lies in a range of from 0.1 % to 5 % (w/v), in particular from 0.2 % to 4.5 % (w/v), in particular from 0.3 % to 4 % (w/v), in particular from 0.4 % to 3.5 % (w/v), in particular from 0.5 % to 3 % (w/v), in particular from 0.6 % to 2.5 % (w/v), in particular from 0.7 % to 2 % (w/v), in particular from 0.8 % to 1.5 % (w/v), in particular from 0.9 % to 1 % (w/v).

All embodiments of the various uses of the sulfated lignin can be combined in any desired way and can be transferred either individually or in any arbitrary combination to the respective other uses, to the described methods, and to the article. Likewise, all embodiments of the various methods can be combined in any desired way and can be transferred either individually or in any arbitrary combination to the respective other methods, to the uses, and to the article.

Finally, all embodiments of the article can be combined in any desired way and can be transferred either individually or in any arbitrary combination to the uses and to the methods.

Further details of aspects of the present invention will be explained in the following making reference to exemplary embodiments and accompanying Figures. In the Figures:

FIG 1A schematically shows the sulfation process of lignin;

FIG 1B schematically shows the synthesis of a hydrogel starting from sulfated lignin;

FIG 2A shows $^{31}$P NMR spectra of bare lignin (BL) as well as of the sulfated lignins SL1 and SL2;

FIG 2B illustrates the surface charge of BL, SL1, and SL2 as determined by zeta potential analysis;

FIG 2C illustrates the relative viability of Vero E6 cells after 48 h exposure to BL, SL1, SL2, or dimethyl sulfoxide (DMSO);

FIG 3A illustrates the swelling capacity analysis of BL, SL1, and SL2 hydrogels;

FIG 3B illustrates the storage modulus of sulfated lignin hydrogels at different lignin concentrations;

FIG 3C illustrates the loss modulus of sulfated lignin hydrogels at different lignin concentrations;

FIG 3D shows the effect of the degree of sulfation on the storage modulus of the sulfated lignin hydrogels;

FIG 3E shows storage modulus plots of SL2 hydrogel after incubation with hyaluronidase and without enzymatic treatment;

FIG 3F shows loss modulus plots of SL2 hydrogel after incubation with hyaluronidase and without enzymatic treatment;

FIG 3G illustrates the effect of enzymatic treatment on the storage modulus of BL, SL1, and SL2 hydrogels;

FIG 3H shows SEM micrographs of bare and sulfated lignin;

FIG 4A shows a quantitative estimation of HSV-1 inhibition by BL, SL1, and SL2 powders;

FIG 4B shows a quantitative estimation of HSV-1 inhibition by BL, SL1, and SL2 hydrogels having different weight percentages of lignin;

FIG 5A shows an estimation of *E. coli growth* based on optical density (OD) measurement of different hydrogel samples; and

FIG 5B    shows a quantitative estimation of *E. coli* inhibition by lignin hydrogels.

The Figures will be explained in the following making reference to exemplary embodiments.

**Sulfated lignin and hydrogel synthesis**

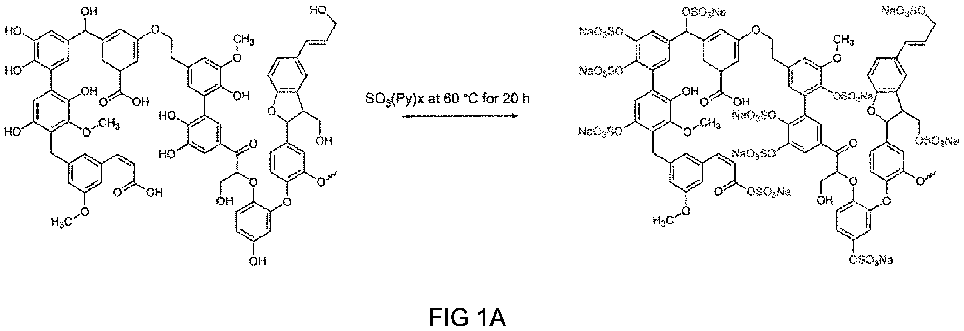

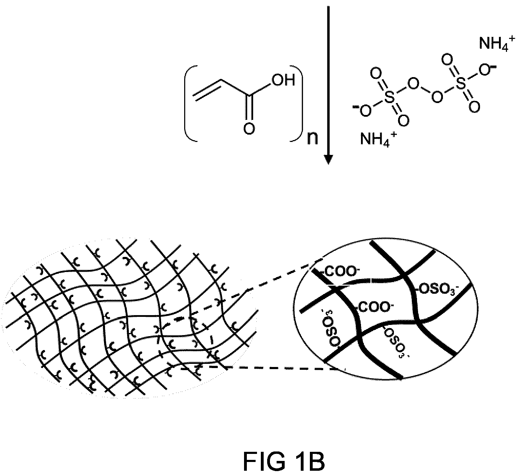

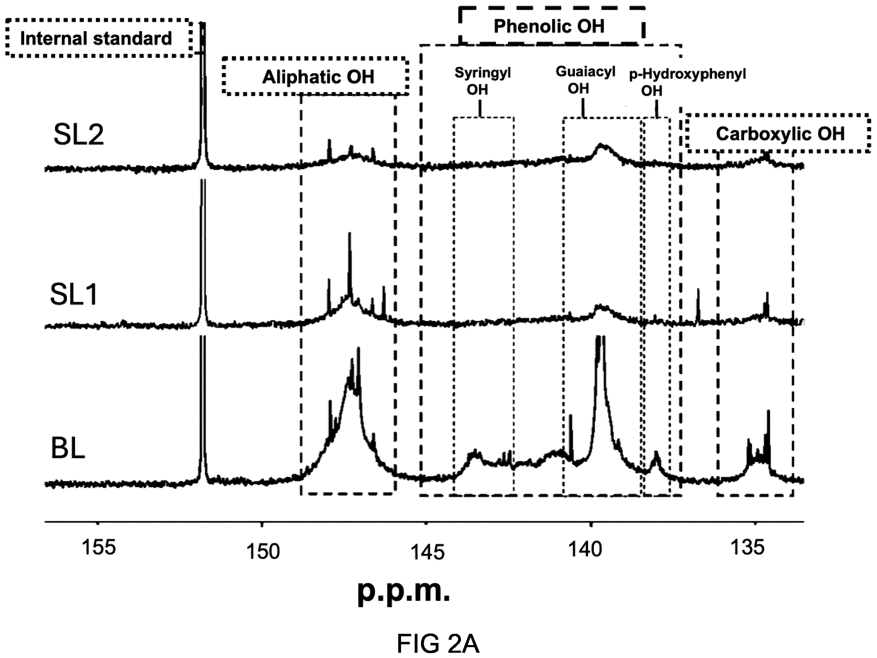

Kraft lignin has been functionalized with sulfate groups to generate sulfated lignin (represented by an exemplary chemical scheme in Figure 1A). The success of functionalization has been confirmed by $^{31}$P NMR analysis (Figure 2A) and elemental analysis. While Figure 1B schematically illustrates a conversion rate of hydroxyl groups to sulfate groups of 100 %, the maximum degree of sulfation achieved was approximately 90 %. Thus, the sulfated lignin comprises non-reacted hydroxyl groups.

The lignin hydrogel was synthesized by copolymerizing sulfated (functionalized) lignin with polyacrylic acid (PAA) using ammonium persulfate (APS) as a free radical initiator (see scheme of Figure 1B). The use of APS as an initiator allows for the efficient initiation of radical polymerization, resulting in the formation of a three-dimensional network structure. This facilitates the crosslinking through ester bond formation between the (remaining) -OH bonds of lignin and -COOH bonds of polyacrylic acid, which leads to the formation of a stable hydrogel network (Figure 1 and 2a). The synthesis process was straightforward and simple, which was carried out at 70 °C for 30 minutes. The synthesized hydrogels were bioinspired from the native mucus and were further evaluated for their physical and biological properties.

The lignin hydrogels were synthesized by copolymerizing functionalized lignin with polyacrylic acid (PAA) using ammonium persulfate (APS) as a free radical initiator. The use of APS as an initiator allows for the efficient initiation of radical polymerization, resulting in the formation of a three-dimensional network structure. This facilitates the crosslinking through ester bond formation between the (remaining) -OH bonds of lignin and -COOH bonds of polyacrylic acid, which leads to the formation of a stable hydrogel network (cf. Figure 1B). The hydrogel synthesis was carried out at 70 °C for 30 minutes. The synthesized hydrogels were further evaluated for their physical and biological properties.

**Physical characterization of functionalized lignin powders**

To assess the impact of the degree of sulfation (DoS) on the physicochemical properties of lignin powder, two types of sulfated lignin (denoted as SL1 and SL2 in the following) were examined and compared with bare lignin (BL). In SL1, approximately three quarter of the available hydroxyl groups have been converted into sulfates. In SL2, nearly 90 % hydroxyl groups have been converted. This conversion was monitored based on the $^{31}$P NMR chemical shifts in the region of 146 to 148 ppm corresponding to aliphatic hydroxyl groups and showing a significant decrease in case of SL1 and SL2 (Figure 2A).

Moreover, chemical shifts were observed in the region of 137 to 145 ppm corresponding to the phenolic groups in lignin (syringyl, guaiacyl and p-hydroxyphenyl). The amount of aliphatic hydroxyl and phenolic units were calculated in BL, SL1, and SL2.[35] The decrease in these groups is paralleled by an increase in sulfates as indicated by elemental analysis, which proves successful sulfation of the lignin, leading to DoS values of approximately 78 % (SL1) and 90 % (SL2).

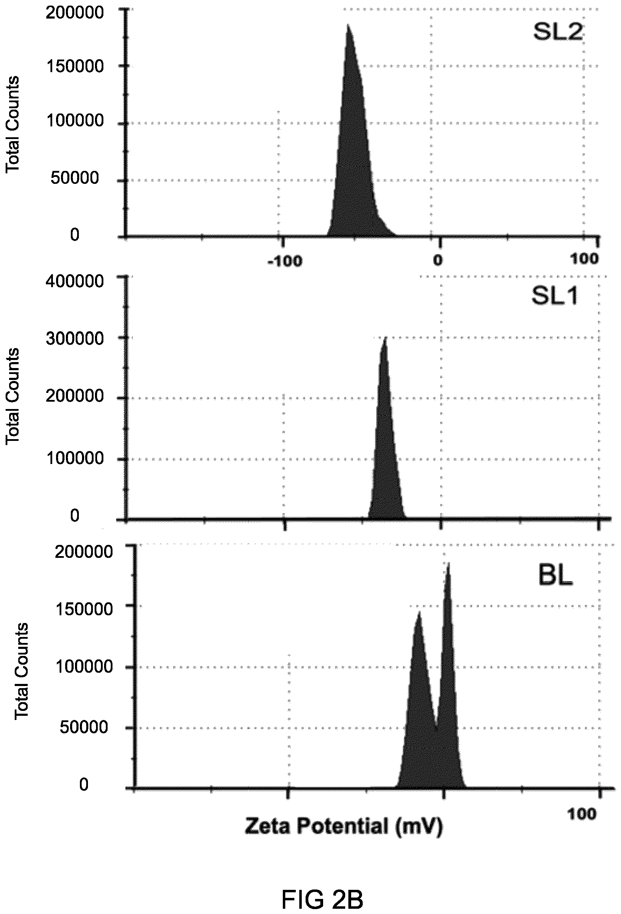

The sulfation process adds negative charges to lignin that increases with the degree of sulfation (Figure 2B). The surface charge of the lignin samples was measured through zeta potential analysis, which is a measure of the electrostatic charge at the surface of particles or molecules in a colloidal dispersion. The surface charge influences the stability and behavior of colloidal systems, such as suspensions and emulsions.[36, 37]

This addition of negatively charged groups to lignin significantly alters its zeta potential. Therefore, the values of zeta potential decrease from -21.9 mV in the case of bare lignin to -35.4 mV (SL1) and -50.2 mV (SL2), respectively (Figure 2B). The observed increase in negative surface charge further supports the results from $^{31}$P NMR and elemental analysis. It can be hypothesized that the increased negative charge density leads to stronger electrostatic repulsion between sulfated lignin particles or molecules. This repulsion prevents them from coming close together and agglomerating, thereby enhancing the stability of colloidal dispersions containing sulfated lignin.

For the determination of the molecular weight distribution of bare and sulfated lignin, size exclusion chromatography (SEC) was performed. The weight average molecular weight ($M_w$) of the bare (unmodified) lignin was deduced to be 7278.6 Da. It was observed that the peak in the chromatogram becomes narrower, and the $M_w$ increases upon sulfation to 9535.5 (SL1) and 8641.3 Da (SL2), respectively. This clearly indicates that the molecular weight distribution of sulfated lignin shifts to larger values and becomes more monodisperse.

These results were further confirmed by particle size distribution analysis of the lignin samples. Further, UV-visible (UV-VIS) spectroscopy displayed no significant shifts of the absorption maxima of lignin (centered at ~ 280 nm) upon sulfation, suggesting that introducing sulfate groups does not significantly alter the electronic transitions that UV-VIS spectroscopy is sensitive to.

To gain further insights into the impact of sulfation on the chemical structure of lignin, Fourie-transform infrared (FTIR) spectra of sulfated lignin and bare (unmodified) lignin were recorded. A sharp peak was observed between the region of 1200 and 1300 $cm^{-1}$, which is, in agreement with previous studies, attributed to sulfate groups (-$SO_3H$).[16, 38] There were also peak shifts observed in the CO stretching mode of lignin occurring from ~ 1020 to ~1100 $cm^{-1}$. Moreover, a sharpening of the OH peak at ~ 3500 $cm^{-1}$ was observed indicating intramolecular hydrogen bonds.

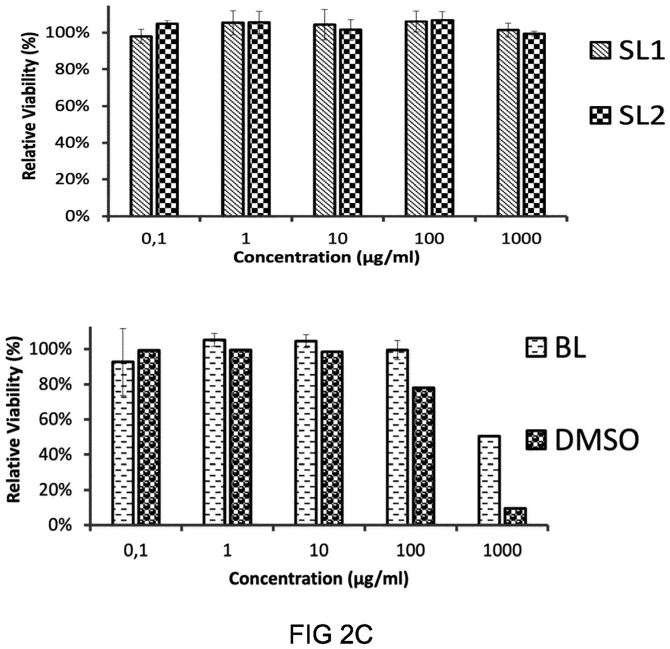

Polymers designed to mimic mucus or to be otherwise used as drug should be biocompatible and safe for use in biological systems without causing harm or triggering an immune response. To investigate the biocompatibility of the sulfated lignin polymers, the viability of VeroE6 cells (as a model for fibroblast cells which are also used for virus propagation) was tested after 48 h of exposure (Figure 2C, left plot). It was observed that cells exposed to SL1 or SL2 showed no reduction in their viability up to a polymer concentration of 1 mg/mL, indicating a high compatibility with Vero E6 cells. The bare lignin showed a decrease in the cell viability at a concentration of 1 mg/mL, which is attributed to the effect of DMSO that had to be added to solubilize bare lignin (Figure 2C, right plot). The addition of DMSO was not necessary for the sulfated lignin samples, as they were completely soluble in water.

Interestingly, the biocompatibility of the sulfated lignin polymer was significantly higher than that of bare lignin. Therefore, it can be concluded that by introducing sulfate moieties into the macromolecular architecture of lignin, the resulting derivatives i) exhibit improved solubility in an aqueous solution, ii) have an enhanced reactivity, making them more amenable to chemical reactions, such as crosslinking, grafting, and polymerization, and iii) have an increased biocompatibility.

**Table 1.** Zeta potential, size, SEC-determined molecular weight as well as the $IC_{50}$ inhibitory constant for the interaction of the lignin with human herpes simplex virus 1 (HSV-1).

| Sample | Particle Size (nm) | Zeta Potential (mV) | Molecular weight, Mw (Da) | $IC_{50}$ values ($\mu$g/ml) | $IC_{50}$ values (nM) |
|---|---|---|---|---|---|
| BL | 309 $\pm$ 16 | -21.9 $\pm$ 2.54 | 7278.6 | 86.8 | 11.9 * $10^3$ |
| SL1 | 216 $\pm$ 9.8 | -28.7 $\pm$ 3.15 | 9535.5 | 2.85 | 298 |
| SL2 | 167 $\pm$ 8 | -35.4 $\pm$ 1.69 | 8641.3 | 0.48 | 55.5 |

## Hydrogel Characterization: Rheology, Enzymatic Stability, and Electron Microscopy

After the detailed analysis of the surface characteristics, the bare and sulfated lignins were subjected to hydrogel synthesis. The parameters for the hydrogel synthesis were designed and optimized carefully to mimic native mucus hydrogels. The high solubility of the sulfated lignin in aqueous medium facilitated the hydrogel synthesis through a sustainable and mild process. Spinnability in hydrogels is their capability of thread-formation arising from non-Newtonian flow. For this, an important prerequisite is the existence of both viscous and elastic properties. Initially, with a lignin concentration of 2 wt %, the obtained hydrogel was rigid and exhibited high viscoelastic properties, and also did not tend to flow within the measured time period. The synthesized lignin hydrogel was also quite elastic as it showed minimal flow upon pressure application and as pressure was removed, it recoiled quickly back to its previous position. However, as the lignin concentration was decreased from 2 wt % to 0.5 wt %, the obtained hydrogel appeared to be viscous with high spinnability, maintaining its network structure and elasticity.

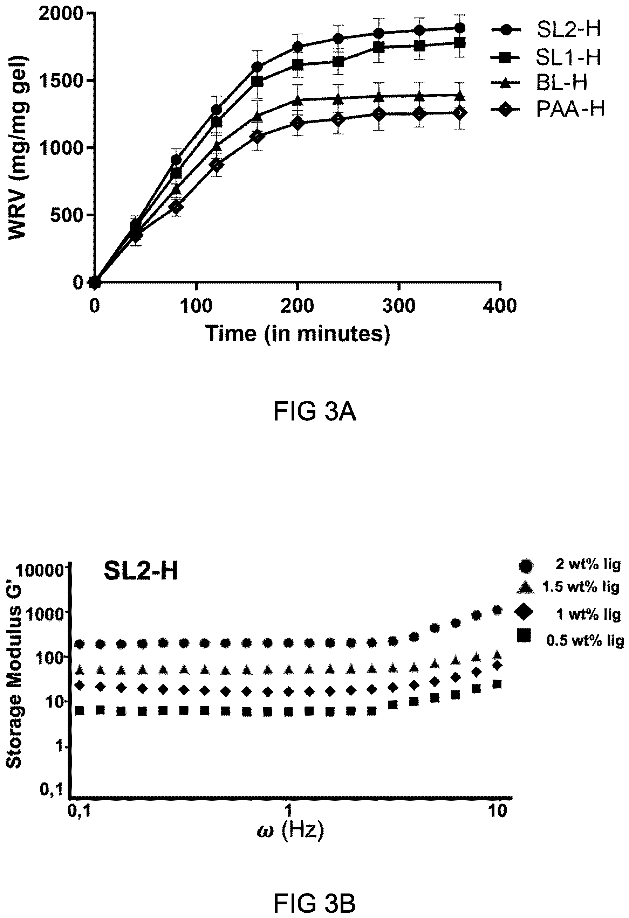

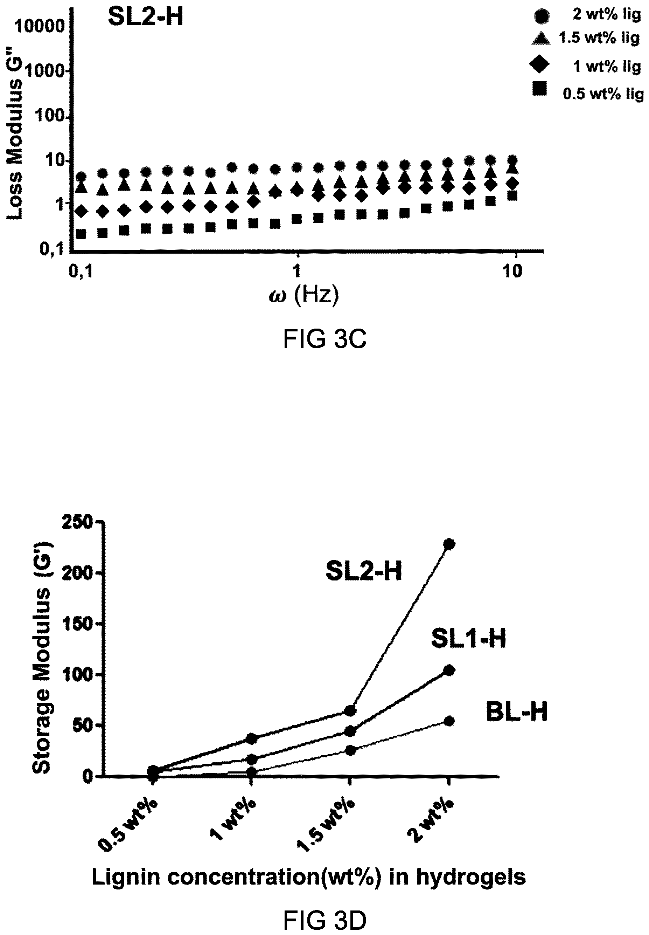

As hydrogels are polymeric materials that can swell in water and retain a significant fraction of water within their structure, the swelling rate is one of the most important properties of hydrogels.[39] To measure the swelling rate of sulfated lignin and bare lignin hydrogels, the profile of swelling capacity versus time of the hydrogel samples was obtained by performing free-absorbency capacity measurements at consecutive time intervals. Firstly, the synthesized hydrogels were subjected to freeze drying and then the completely dried hydrogels were kept inside water for swelling. It was deduced that the free swell water retention value (WRV) of the sulfated lignin hydrogel was approximately 1600, which was reduced to -1454 in the case of bare lignin hydrogels (Figure 3A). However, even the bare lignin hydrogel exhibits high water absorption abilities.

The mechanical properties of a substance are indicated by its viscoelastic properties which can be evaluated through oscillatory rheology experiments. Mucus hydrogels are viscoelastic and sticky, making them effective at trapping and immobilizing foreign particles and pathogens. Viruses, bacteria, and other microorganisms can become entangled in the mucus matrix, preventing them from reaching and infecting the underlying cells. For a hydrogel to mimic native mucus, its storage modulus and loss modulus should lie in the range of 1-10 Pa and 0.1 to 1 Pa, respectively.[40, 41]

Initially, to establish the linear viscoelastic region, the entire series of produced hydrogels was subjected to strain-sweep tests. In addition, consecutive oscillatory rheology experiments were conducted in this region. The storage modulus and loss modulus were deduced as a function of the radial frequency $\omega$. All the experiments were conducted at 25 °C. The rheological behaviour of hydrogels formed using bare lignin (BL-H) or sulfated lignins (SL1-H and SL2-H, respectively) is shown in Figures 3B to 3D. All synthesized hydrogels exhibited significant elastic-dominant behaviour, thus proving the stability of the crosslinks. The concentration of lignin during hydrogel formation was optimized to modulate the rheological properties of the developed hydrogel. Varying the concentration of lignin between 0.5 % and 2 % revealed a significant impact on the elastic and viscous modulus (G' and G", respectively) of the resulting hydrogel (cf. Table 2). Upon careful observation, it was found that upon sulfation of the lignin hydrogels the storage modulus increased by roughly two orders of magnitude, which indicates an increase in cross-linking density in the sulfated lignin hydrogels. This finding was used to optimize the synthesis parameters to achieve the G' and G" values of the order of native mucus.

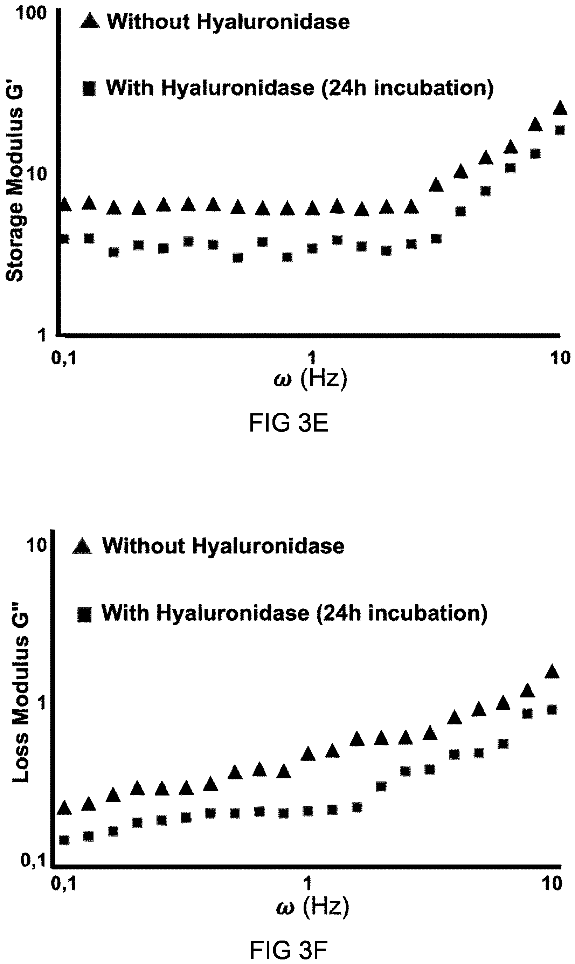

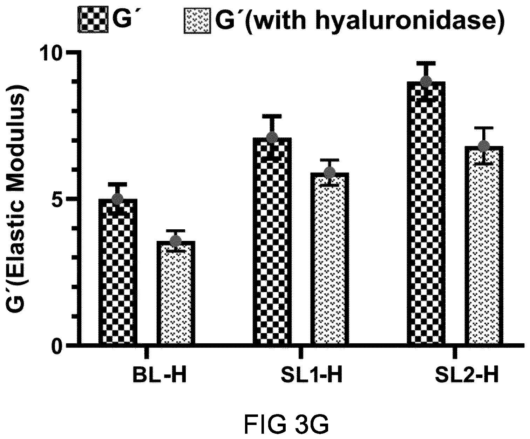

Further, the synthesized hydrogels were tested for their stability against enzymatic cleavage by hyaluronidase. Hyaluronidase is an enzyme present in synovial fluid, which is responsible for lubrication of joints and acts as a shock absorber. It helps reduce friction between bones and cartilage during joint movement, contributing to joint health and mobility. In order to test the stability of the synthesized hydrogel in presence of hyaluronidase, hydrogels were incubated with the enzyme at 37 °C for 30 hours, followed by evaluation of hydrogel rheology and by measuring changes of storage and loss modulus. It was observed that the viscosity of all the hydrogels (BL-H, SL1-H, and SL2-H) were affected by hyaluronidase incubation; however, the hydrogels still maintained storage modulus values between 1 to 10 Pa up to a frequency of 5 Hz (Figures 3E and 3F). Similarly, after enzyme treatment the loss moduli of the BL-H, SL1-H, and SL2-H were in the range of 0.1 to 1 Pa, which highly resembles native mucus (Figure 3G). Further incubation of the hydrogels with hyaluronidase did not affect their rheology and physical structure or appearance. This analysis concludes that the synthesized hydrogels are highly stable against enzymatic degradation by natural enzymes, such as hyaluronidase.

**Table 2.** Storage (G') and loss (G") modulus values of lignin hydrogels as a function of lignin concentration at a radial frequency ($\omega$) of 1 Hz at 37 °C.

| Lignin Concentration (weight %) | Storage Modulus (G') | | | Loss Modulus (G") | | |
|---|---|---|---|---|---|---|
| | BL-H | SL1-H | SL2-H | BL-H | SL1-H | SL2-H |
| 2 | 55.56 | 105.6 | 229.1 | 6.6 | 5.5 | 6.8 |
| 1.5 | 26.63 | 45 | 64.9 | 2.14 | 2.2 | 2.4 |
| 1 | 5.82 | 17.3 | 37.8 | 0.6 | 1.9 | 1.52 |
| 0.5 | 0.13 | 4.9 | 6.7 | 0.03 | 0.8 | 0.48 |

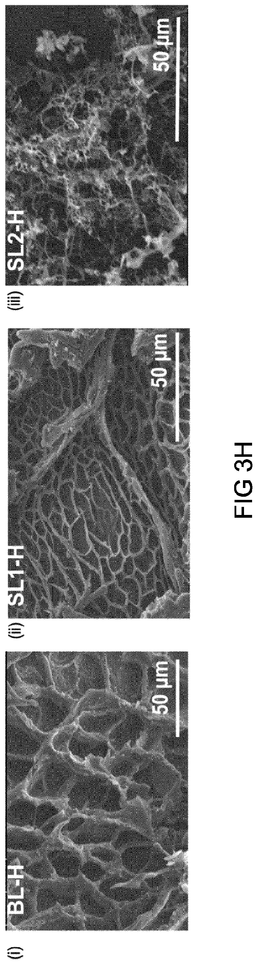

After the rheological analysis, the lignin hydrogels were subjected to scanning electron microscopy (SEM) analysis, which provided valuable insights into the surface morphology and internal structure of the hydrogel. In the obtained SEM images of SL1 and SL2 (1 w/v % lignin), the surface of the sulfated lignin hydrogels appeared to be highly porous, with pore sizes ranging from 800 nm to 2 $\mu$m (SL2; Figure 3H, plot (iii)), or from 8 $\mu$m to 10 $\mu$m (SL1; Figure 3H, plot (ii)), respectively. In case of bare lignin (BL; Figure 3H, plot (i)) the pore size was in the order of 25 $\mu$m. This porous morphology of SL1 and SL2 clearly resembled the SEM images of native mucus. The measurements indicate that increasing the DoS causes the pore size distribution to shift to smaller values (i.e., a decreasing pore size with increasing degree of functionalization).

This observation was made at different lignin weight concentrations of the hydrogels, pointing towards the interesting possibility of tuning the pore size distribution of lignin hydrogels using the appropriate degree of functionalization, *i.e.,* sulfation. There are few reports on tuning the pore size distribution of hydrogels using variable concentrations of the precursor,[42] however the effect of the degree of functionalization of precursor molecule on the mesh size of the hydrogels is still an unexplored area. This interesting finding can be utilized in different areas to tune the porosity of hydrogels by varying the degree of functionalization of the precursor molecule.

**Biological Studies and Pathogen Binding**

Inhibiting virus entry into the host cells at an early stage of infection is a promising concept of infection prevention and treatment. Due to the presence of glycan receptors, HSV-1 binds to the heparan sulfates on the host cell surface.[9, 43] This binding is prevented by the presence of lignin functionalized with sulfate moieties, which multivalently inhibits the virus due to the affinity of the glycan receptors to sulfates on lignin. After a thorough analysis of the chemical structure and physical properties of the sulfated lignin polymers, their antiviral activity against HSV-1 was studied.

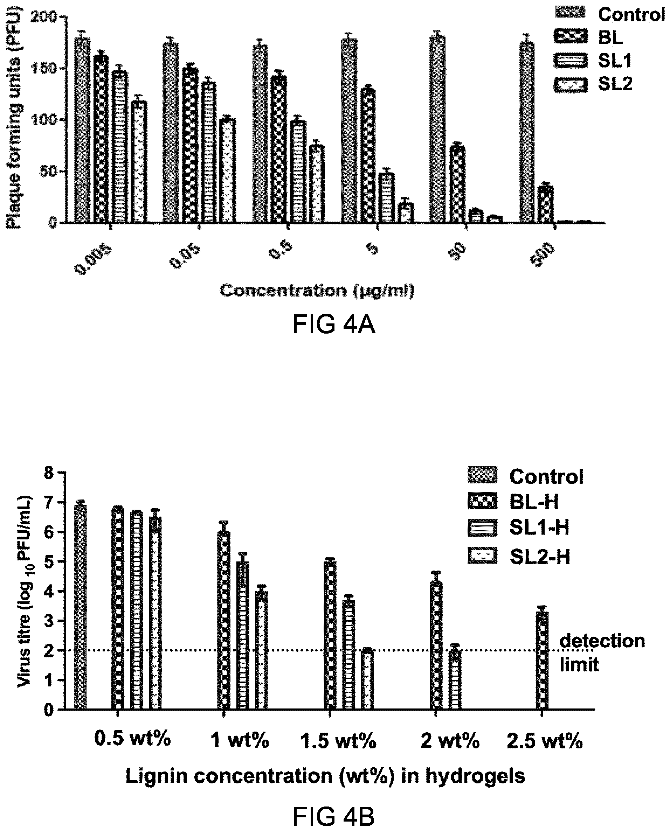

For this purpose, a plaque reduction assay was performed, the goal of which is to determine the concentration of an antiviral compound required to reduce viral plaque formation by a certain percentage. The lignins BL, SL1 and SL2 were tested in aqueous solutions. The samples at different concentrations were mixed with an HSV-1 solution (200 PFU) for 45 minutes at room temperature. Then, the mixture was allowed to infect cells for 45 minutes at room temperature. After washing, the cells were infected with overlay medium for 48 h at 37 °C. As the virus has been modified with the green fluorescent protein (GFP) gene, infected cells expressed GFP and hence exhibited green fluorescence. The cells were observed in the 4',6-diamidino-2-phenylindole (DAPI; blue) and GFP (green) channels. This enabled a quantification of the fraction of infected cells (GFP channel) across the entire cell ensemble (DAPI channel, staining the nucleus of cells). There were no plaques in case of the sulfated lignin, which gives proof of complete inhibition of the virus. Extracting dose-dependent inhibition curves from this data indicated that increasing the degree of sulfation leads to higher inhibition (Figure 4A). It could be observed that SL2 shows the highest inhibition, followed by SL1 and BL. The $IC_{50}$ values of BL, SL1, and SL2 were found to be 86.8, 2.9, and 0.5 μg/ml respectively (summarized above in Table 1).

Further, the HSV-1 binding to lignin-based hydrogels was investigated in a related assay. For this, the BL-H, SL1-H, and SL2-H hydrogels were incubated with HSV-1 for 1 h at 37 °C. Afterwards, the number of infectious viruses in the supernatant was titrated on Vero E6 cells via plaque assay. The reduced virus titer in the supernatant reveals the binding of the hydrogels with the HSV-1 (represented in Figure 4B) and the $IC_{50}$ values are summarized in Table 1 above. The sulfated lignin hydrogels exhibited much higher retention abilities than their non-sulfated counterparts. Through the glycoprotein C (gC), HSV uses electrostatic interactions to bind with the heparan sulfates on the host cells.[43] As the SL1-H and SL2-H present more sulfate groups on their surface, there is more virus binding compared to the BL-H, both in the case of hydrogels and hydrogel components, *i.e.,* lignin. The concentration of sulfated lignin played an important role in the binding ability of the hydrogels, as the binding and inhibition of the viruses increased with an increase in the lignin concentrations. It was observed that there was complete adsorption of the HSV-1 at 1.5 wt % concentration of SL 1-H hydrogels and 1 wt % of SL2-H hydrogels. It can also be correlated with the increased cross-linking density and porous network of SL2-H as compared to SL1 and BL. Moreover, the hydrogels with better viscoelastic properties exhibited better binding towards the virus particles. The SEM and rheology results perfectly complied with the HSV-1 binding studies, hence, proving that SL2-H is the best candidate for virus binding and inhibition.

Interestingly, the impact of DoS on the inhibition efficacy was much more pronounced for lignin-based hydrogels than for (non-crosslinked) lignin: While addition of sulfates to BL improved the inhibition concentration by two orders of magnitude (BL vs. SL2, Figure 4A), a much larger reduction of more than four orders of magnitude was found for the lignin-based hydrogels (BL-H vs. SL2-H, Figure 4B), indicating that hydrogel formation indeed offers a merit in comparison to a direct application of the sulfated lignins. This puzzling observation might be attributed to an optimized 3D presentation of sulfate groups within the hydrogel and motivates further investigation on the impact of hydrogel structure and functionalization on pathogen retention.

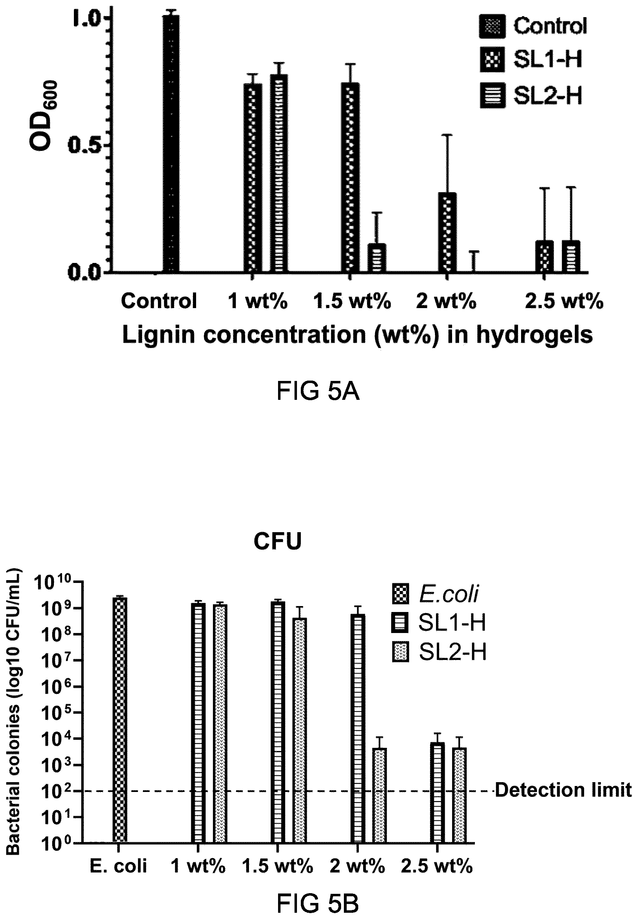

In addition, the compounds were also tested for their activity against *E. coli* (Figures 5A and 5B) and influenza A virus, allowing to assess a potential broad-spectrum antimicrobial activity of the lignin-based hydrogels. Interestingly, the sulfated lignin hydrogels were effective against influenza A as well. The effect was, however, less pronounced in comparison to HSV-1. This inhibition effect could be attributed to the surface adsorption or adherence of influenza A towards the lignin hydrogels. In contrast, the developed hydrogels were highly effective against *E. coli* bacteria, as indicated by a strong adsorption of bacteria from solution (Figure 5A) and a reduction of colony forming units by more than 5 orders of magnitude (Figure 5B).

As the inhibition is more prominent only in the case of the sulfated lignin hydrogels (SL1-H and SL2-H), it is possible that the acidic behavior of the sulfated lignin hydrogels (due to the release of sulfate ions) can also be responsible for the inhibitory behavior of hydrogels along with the surface adherence of the pathogens. Moreover, the intrinsic antimicrobial activity of lignin can also play a certain role in the inhibitory activity of the lignin hydrogels. These experiments conclude that the sulfated lignin hydrogels can be applied for a broad spectrum of pathogens and hence, increases their applicability also as mucus-mimicking hydrogels.

**Experimental Details**

*Sulfation of lignin*: For the sulfation of lignin (Sigma-Aldrich/Merck), sulfur trioxide pyridine complex was used. Lignin (400 mg) was mixed with sulfur trioxide pyridine complex (1.5 eq. w/w) in dry DMF (20 mL) at 60 °C for 20 h under argon atmosphere. After the reaction time was over, the pH value was increased to pH 10 by the addition of sodium hydroxide solution (0.3 mol $L^{-1}$). The product was dialyzed against sodium hydroxide solution (0.3 mol $L^{-1}$), 10 wt % NaCl and then water to remove the unreacted components.

*Characterization of the sulfated lignin:* The yield of the sulfation was analyzed by elemental analysis. Further, the molecular weight distribution of the bare and sulfated lignin (8 mg/mL) was determined by means of gel permeation chromatography (GPC) coupled to a refractive index detector (R!) for obtaining the complete distribution ($M_n$, $M_p$, $M_w$, dispersity) of molecular weight with a GPC device comprising an Agilent 1100 solvent delivery system with pump, manual injector, and an Agilent differential refractometer. The hydrodynamic diameter was measured by dynamic light scattering at a concentration of 1 mg $mL^{-1}$ in PBS buffer using Zetasizer Nano series ($\lambda$ = 532 nm) from Malvern Panalytical. Before the measurement, all samples were subjected to sonication for 15 minutes in a bath sonicator. Temperature equilibration was done for 1 min at 25 °C. The measurements were performed for ten scans each 15 s and the mentioned values result from at least three measurements. The surface charge was investigated by zeta-potential measurement with Zetasizer Nano series ($\lambda$ = 532 nm) using folded capillary zeta cells (DTS 1070) from Malvern Panalytical (Kassel, Germany).

*Dynamic Light Scattering and Zeta Potential Analysis*: For measuring the hydrodynamic diameter and the surfaces charge, dynamic light scattering (DLS) was performed at a concentration of 0.3 mg $mL^{-1}$ in deionized water using Zetasizer Nano series from Malvern Panalytical (Kassel, Germany). Disposable cuvettes (ZEN0040) made of polystyrene were used from Brand (Wertheim, Germany). All the samples were sonicated for 15 minutes before the measurement. Temperature equilibration was done at 25 °C for 1 min. The measurements were performed for ten scans each 15 s in back scattering mode (173°). The average values resulting from at least three measurements were recorded. For the surface charge analysis, folded capillary zeta cells (DTS 1070) were procured from Malvern Panalytical (Kassel, Germany). The average values resulting from five measurements with ten scans (each 15 s) were recorded to obtain the zeta potential for SL2, SL1 and BL.

*Cell Viability Test*: Vero E6 cells (Leibniz Institute DSMZ - German Collection of Microorganisms and Cell Cultures GmbH) were routinely cultured in Dulbecco's Modified Eagle Medium (DMEM) (#31966-021, Gibco/Thermo Fisher Scientific) supplemented with 10 % fetal bovine serum (FBS) (#10270-106, Gibco/Thermo Fisher Scientific) and 1 % Penicillin-Streptomycin (#P4333-100ML, Sigma-Aldrich/Merck) at 37 °C and 5 % $CO_2$. For the viability test, 100 $\mu$L per well of a 10000 cells/mL suspension in the medium was seeded into white 96-well plates and incubated overnight. The next day, the test compounds were solubilized to a concentration of 10 mg/mL in Milli-Q $H_2O$ or DMSO to a concentration of 10 mg/mL. Then, the compounds were further diluted in cell medium as a 10-fold serial dilution starting with the highest concentration of 1 mg/mL. The supernatant of the cells was removed and replaced with 100$\mu$L per well of the different compound dilutions in the medium. After 48 h of incubation, the supernatant was removed and replaced with 50$\mu$! per well fresh medium and 50$\mu$L per well CellTiter-Glo® Reagent, which was prepared according to the instructions of the manufacturer (CellTiter-Glo® Luminescent Cell Viability Assay, #G7570, Promega). Well contents were mixed for 2 minutes on a shaker, incubated at room temperature for 30 min and the luminescent signal was measured in a SPARK® multimode microplate reader (Tecan). All tests were prepared 3 times independently in duplicates. Average values of the test wells were calculated, divided by the average values of untreated cells and expressed as % viability +/standard deviation.

*Synthesis of lignin hydrogels:* The synthesis of lignin-based hydrogels was carried out using mild procedures by the inclusion of various optimized conditions. The aqueous sulfated lignin solutions were prepared in various concentrations (0.5 to 2 % w/v) for optimization of the lignin concentration. This was followed by the addition of polyacrylic acid aqueous solution (2.8 % w/v) to the reaction mixture and stirring at room temperature at a speed of 300 rpm for 5 min. Finally, ammonium persulfate (APS) (3 % w/v) was added to the reaction mixture for the initiation of free radical generation, which mediated the cross-linking and co-polymerization between lignin and polyacrylic acid through formation of ester linkages. The reaction vials were then transferred to an incubator and left undisturbed at 70 °C for 20 min. After cooling down to room temperature, the lignin-based hydrogels were dialyzed with PBS for ~12 h using a 1 kDa membrane. This facilitated the removal of unreacted APS and other contaminants.

*Analysis of the swelling capacity of hydrogels:* The hydrogels were immersed in deionized water. At each time interval, swelling gels were withdrawn from the solution and weighed out after removing the excess liquid from the gel surface. The excess water (or salt solution) was removed by filtration. The free swell water retention value (WRV), a measure of the dynamic water absorption properties of the gel, was calculated using the following equation:

$$WRV = (Wt - Wd)/Wd \times 100$$

where Wt is the weight of the wet gel at time t and Wd is the weight of the dried gel.

*Enzyme degradation studies:* The filtered enzyme solution was aliquoted and frozen at -20 °C. Then, 500 $\mu$l of hydrogel were pipetted into 2-ml Eppendorf tubes using cut tips (200 $\mu$l), also known as 'wide bore'. This was followed by the addition of 100 $\mu$l of the enzyme solution/PBS to the tubes and thorough mixing by pipetting. The viscosity of the samples was measured directly, after 24 hours at 37 °C. The samples were temporarily stored at 37 °C without shaking.

*Virus propagation and titration:* HSV-1-GFP virus was kindly provided by Prof. Dr. Benedikt Kaufer and Prof. Dr. Klaus Osterrieder from Freie Universität Berlin. The virus was propagated on Vero E6 cells. Briefly speaking, the Vero E6 cells were seeded in T75 flask and showed >90 % confluency for the infection. Then the cells were infected by HSV-1-GFP at MOI 0.1 for 2 days in DMEM medium (with 10 % FBS, Pen/strep). Then, the cell culture supernatant was collected and centrifuged at 1000 rpm for 10 min. The supernatant after centrifugation was aliquoted and stored at -80 °C. The virus stock was titrated by plaque assay on Vero E6 cells using Minimum Essential Medium (MEM) containing either 0.9 % methycellulose or 0.75 % Avicel (90 % microcrystalline cellulose + 10 % carboxymethycellulose) as the overlay. After having been fixed, the plaques could be counted either under a fluorescent microscope or by crystal violet staining.

*Plaque reduction assay:* The compounds were diluted 10-fold in 100 $\mu$L infection medium. Then, 100 $\mu$L of approx. 4000PFU/ml HSV-1-GFP dilution were added to the sample and incubated for 45min at room temperature. Then, 100 $\mu$L of the virus was added onto Vero E6 cells and incubated at room temperature with moderate shaking for 45 min. Finally, the cells were washed once with PBS and then infected with the overlay medium for 2-4 days for plaque development. The plaques were counted as mentioned above.

*Virus binding assay:* The samples (5 $\mu$L each) were subjected to incubation with 100$\mu$L HSV-1-GFP solution (approx. $1 \times 10^6$ PFU/mL) for 1 hour. Then, the supernatant was collected and titrated by plaque assay on Vero E6 cells as described above.

*Fluorescence microscopy*: After having been fixed by 4 % formaldehyde for 30 min, the cells were permeabilized with 0.1 % Triton-X-100 in PBS at room temperature for 10 minutes, followed by washing once with PBS. Afterwards, DAPI (10 $\mu$g/ml) was added to stain the cell nuclei for 10 minutes. The cells were then washed with PBS, and then subjected to fluorescence microscopy. The images were acquired at 10x objective in the DAPI and GFP channels with a fluorescence microscope (Axio, Zeiss, Germany).

*In vitro antibacterial activity via growth curves and CFU assays:* The K-12 derivative *E. coli* AR3110 was streaked out on LB agar from frozen stock. Single-colony was used to create the liquid culture that was grown to an $OD_{600}$ of 0.600-0.800 at 37 °C in LB media under shaking. The culture was then diluted in LB to a final $OD_{600}$ concentration of 0.01, and 100 $\mu$L of this culture were added to 100 $\mu$L of UV-sterilized hydrogel of varying concentrations in a 96-well plate (round bottom, Sarstedt). Each well was thoroughly mixed by pipetting. The lid of the 96-well plate was coated in anti-fog solution (0.05 % TritonX-100, 20 % Ethanol in water[44] and allowed to completely dry under a clean bench hood. The 96-well plate closed with lid was placed in the Agilent BioTek Epoch 2 Microplate Spectrophotometer and allowed to grow for 20 hours at 37 °C with continuous shaking (double orbital). Measuring of $OD_{600}$ was done in 15 min intervals. The $OD_{600}$ after 20 hours was used for graph generation. Three biological repeats were performed consisting each of three technical repeats. Error bars represent $\pm$ standard deviation (SD). Statistical analysis was executed using the GraphPad Prism software (GraphPad Software, Inc., San Diego, CA).

Colony forming units (CFUs) were then preformed after the 20-hour growth curve using the resultant culture as described in Maan *et al.*[45] Briefly, the culture was transferred to a 96-well plate and a serial dilution from $10^0$ to $10^{-7}$ was performed in DPBS. 20 $\mu$L of varying dilutions were spotted onto LB agar plates and allowed to dry completely in clean bench hood. Plates were incubated overnight at 37 °C and CFUs were then counted. Three biological repeats were performed consisting each of three technical repeats. Error bars represent $\pm$ SD. Statistical analysis was executed using the GraphPad Prism software (GraphPad Software, Inc., San Diego, CA).

## Conclusion

Lignin is a promising scaffold for the large-scale synthesis of biocompatible mucus-mimicking hydrogels that exhibit antimicrobial activity. Sulfating lignin is found to significantly improve its water solubility and its interaction with sulfate-binding viruses, such as HSV-1 , which increases with increasing degree of sulfation. Tuning the concentration and degree of sulfation of the lignin in the hydrogel enabled to form lignin-based hydrogels that resembled native mucus with respect to the presentation of virus attachment factors and rheological properties. SEM analysis revealed that increasing the degree of sulfation decreased the mesh-size distribution by orders of magnitude, which provides a novel and interesting means to fine-tune virus-hydrogel interactions. At the highest degree of sulfation (conversion of ~ 90 % of the available hydroxyl groups), the mesh-size distribution is in the order of native mucus (hundreds of nm). The corresponding hydrogel shows a very efficient infection inhibition, as indicated by a reduction in plaque or colony forming units by more the 4 orders of magnitude. These experiments demonstrated that forming hydrogels from pathogen-binding inhibitors can provide a substantial benefit in infection inhibition (attributed here to an improvement in pathogen sequestration in comparison to the non-crosslinked inhibitor). Although the lignins were functionalized only with sulfates, the high abundance of hydroxyl groups in lignin will enable a feasible extension toward further pathogen attachment factors.

**List of references cited in the preceding sections or found otherwise to be relevant**

1. Beaucamp, A.; Muddasar, M.; Amiinu, I. S.; Leite, M. M.; Culebras, M.; Latha, K.; Gutiérrez, M. C.; Rodriguez-Padron, D.; del Monte, F.; Kennedy, T. Lignin for energy applications-state of the art, life cycle, technoeconomic analysis and future trends. Green Chemistry 2022.

2. Ji, D.; Park, J. M.; Oh, M. S.; Nguyen, T. L.; Shin, H.; Kim, J. S.; Kim, D.; Park, H. S.; Kim, J. Superstrong, superstiff, and conductive alginate hydrogels. Nature Communications 2022, 13 (1), 3019.

3. Bej, R.; Haag, R. Mucus-Inspired Dynamic Hydrogels: Synthesis and Future Perspectives. Journal of the American Chemical Society 2022, 144 (44), 20137-20152. DOI: 10.1021/jacs.1c13547.

4. Leal, J.; Smyth, H. D. C.; Ghosh, D. Physicochemical properties of mucus and their impact on transmucosal drug delivery. Int J Pharm 2017, 532 (1), 555-572. DOI: 10.1016/j.ijpharm.2017.09.018 From NLM.

5. McGuckin, M. A.; Lindén, S. K.; Sutton, P.; Florin, T. H. Mucin dynamics and enteric pathogens. Nature Reviews Microbiology 2011, 9 (4), 265-278. DOI: 10.1038/nrmicro2538.

6. Yang, S.; Duncan, G. A. Synthetic mucus biomaterials for antimicrobial peptide delivery. Journal of Biomedical Materials Research Part A 2023.

7. Zanin, M.; Baviskar, P.; Webster, R.; Webby, R. The Interaction between Respiratory Pathogens and Mucus. Cell Host & Microbe 2016, 19 (2), 159-168. DOI: https://doi.org/10.1016/j.chom.2016.01.001.

8. Lieleg, O.; Lieleg, C.; Bloom, J.; Buck, C. B.; Ribbeck, K. Mucin Biopolymers As Broad-Spectrum Antiviral Agents. Biomacromolecules 2012, 13(6), 1724-1732. DOI: 10.1021/bm3001292.

9. Sharma, A.; Thongrom, B.; Bhatia, S.; von Lospichl, B.; Addante, A.; Graeber, S. Y.; Lauster, D.; Mall, M. A.; Gradzielski, M.; Haag, R. Polyglycerol-Based Mucus-Inspired Hydrogels. Macromolecular Rapid Communications 2021, 42 (20), 2100303.

10. Yan, H.; Melin, M.; Jiang, K.; Trossbach, M.; Badadamath, B.; Langer, K.; Winkeljann, B.; Lieleg, O.; Hong, J.; Joensson, H. N. Immune-Modulating Mucin Hydrogel Microdroplets for the Encapsulation of Cell and Microtissue. Advanced Functional Materials 2021, 31 (42), 2105967.

11. Abdel Aziz, M. H.; Mosier, P. D.; Desai, U. R. Identification of the site of binding of sulfated, low molecular weight lignins on thrombin. Biochemical and Biophysical Research Communications 2011, 413 (2), 348-352. DOI: https://doi.org/10.1016/j.bbrc.2011.08.102.

12. Afewerki, S.; Wang, X.; Ruiz-Esparza, G. U.; Tai, C.-W.; Kong, X.; Zhou, S.; Welch, K.; Huang, P.; Bengtsson, R.; Xu, C.; et al. Combined Catalysis for Engineering Bioinspired, Lignin-Based, Long-Lasting, Adhesive, Self-Mending, Antimicrobial Hydrogels. ACS Nano 2020, 14 (12), 17004-17017. DOI: 10.1021/acsnano.0c06346.

13. Mennani, M.; Kasbaji, M.; Benhamou, A. A.; Boussetta, A.; Mekkaoui, A. A.; Grimi, N.; Moubarik, A. Current approaches, emerging developments and functional prospects for lignin-based catalysts-Review. Green Chemistry 2023.

14. Wan, Y.; He, J.; Zhang, Y.; Chen, E. Y. X. One-Step Synthesis of Lignin-Based Triblock Copolymers as High-Temperature and UV-Blocking Thermoplastic Elastomers. Angewandte Chemie 2022, 134 (8), e202114946.

15. Malyar, Y. N.; Kazachenko, A. S.; Vasilyeva, N. Y.; Fetisova, O. Y.; Borovkova, V. S.; Miroshnikova, A. V.; Levdansky, A. V.; Skripnikov, A. M. Sulfation of wheat straw soda lignin: Role of solvents and catalysts. Catalysis Today 2022, 397-399, 397-406. DOI: https://doi.org/10.1016/j.cattod.2021.07.033.

16. Levdansky, A. V.; Vasilyeva, N. Y.; Malyar, Y. N.; Kondrasenko, A. A.; Fetisova, O. Y.; Kazachenko, A. S.; Levdansky, V. A.; Kuznetsov, B. N. An Efficient Method of Birch Ethanol Lignin Sulfation with a Sulfaic Acid-Urea Mixture. Molecules 2022, 27(19), 6356.

17. Zhang, C.; Shen, X.; Jin, Y.; Cheng, J.; Cai, C.; Wang, F. Catalytic strategies and mechanism analysis orbiting the center of critical intermediates in lignin depolymerization. Chemical Reviews 2023, 123 (8), 4510-4601.

18. Sharma, V.; Tsai, M.-L.; Nargotra, P.; Chen, C.-W.; Sun, P.-P.; Singhania, R. R.; Patel, A. K.; Dong, C.-D. Journey of lignin from a roadblock to bridge for lignocellulose biorefineries: A comprehensive review. Science of The Total Environment 2023, 861, 160560.

19. Sethupathy, S.; Morales, G. M.; Gao, L.; Wang, H.; Yang, B.; Jiang, J.; Sun, J.; Zhu, D. Lignin valorization: Status, challenges and opportunities. Bioresource Technology 2022, 347, 126696.

20. Liu, M.; Han, B.; Dyson, P. J. Simultaneous generation of methyl esters and CO in lignin transformation. Angewandte Chemie International Edition 2022, 61 (40), e202209093.

21. Agustiany, E. A.; Rasyidur Ridho, M.; Rahmi DN, M.; Madyaratri, E. W.; Falah, F.; Lubis, M. A. R.; Solihat, N. N.; Syamani, F. A.; Karungamye, P.; Sohail, A. Recent developments in lignin modification and its application in lignin-based green composites: A review. Polymer Composites 2022, 43 (8), 4848-4865.

22. Santo Pereira, A. d. E.; de Oliveira, J. L.; Savassa, S. M.; Rogério, C. B.; de Medeiros, G. A.; Fraceto, L. F. Lignin nanoparticles: New insights for a sustainable agriculture. Journal of Cleaner Production 2022, 345, 131145.

23. Hatakeyama, H.; Hatakeyama, T. Lignin Structure, Properties, and Applications. Advances in Polymer Science 2009, 232, 1-63.

24. Liu, X.; Inda, M. E.; Lai, Y.; Lu, T. K.; Zhao, X. Engineered living hydrogels. Advanced Materials 2022, 34 (26), 2201326.

25. Yang, D. Recent advances in hydrogels. ACS Publications: 2022; Vol. 34, pp 1987-1989.

26. Blache, U.; Ford, E. M.; Ha, B.; Rijns, L.; Chaudhuri, O.; Dankers, P. Y.; Kloxin, A. M.; Snedeker, J. G.; Gentleman, E. Engineered hydrogels for mechanobiology. Nature Reviews Methods Primers 2022, 2 (1), 98.

27. Joonaki, E.; Hassanpouryouzband, A.; Heldt, C. L.; Areo, O. Surface chemistry can unlock drivers of surface stability of SARS-CoV-2 in a variety of environmental conditions. Chem 2020, 6 (9), 2135-2146.

28. Campbell, K. T.; Wysoczynski, K.; Hadley, D. J.; Silva, E. A. Computational-based design of hydrogels with predictable mesh properties. ACS biomaterials science & engineering 2019, 6 (1), 308-319.

29. Connolly, S. A.; Jardetzky, T. S.; Longnecker, R. The structural basis of herpesvirus entry. Nature reviews Microbiology 2021, 19 (2), 110-121.

30. Pouyan, P.; Nie, C.; Bhatia, S.; Wedepohl, S.; Achazi, K.; Osterrieder, N.; Haag, R. Inhibition of Herpes Simplex Virus Type 1 Attachment and Infection by Sulfated Polyglycerols with Different Architectures. Biomacromolecules 2021, 22 (4), 1545-1554.

31. Thongrom, B.; Sharma, A.; Nie, C.; Quaas, E.; Raue, M.; Bhatia, S.; Haag, R. Scaffold Flexibility Controls Binding of Herpes Simplex Virus Type 1 with Sulfated Dendritic Polyglycerol Hydrogels Fabricated by Thiol-Maleimide Click Reaction. Macromolecular Bioscience 2022, 22 (5), 2100507.

32. Ahmadi, V.; Nie, C.; Mohammadifar, E.; Achazi, K.; Wedepohl, S.; Kerkhoff, Y.; Block, S.; Osterrieder, K.; Haag, R. One-pot gram-scale synthesis of virucidal heparin-mimicking polymers as HSV-1 inhibitors. Chemical Communications 2021, 57 (90), 11948-11951.

33. Nie, C.; Pouyan, P.; Lauster, D.; Trimpert, J.; Kerkhoff, Y.; Szekeres, G. P.; Wallert, M.; Block, S.; Sahoo, A. K.; Dernedde, J. Polysulfates Block SARS-CoV-2 Uptake through Electrostatic Interactions. Angewandte Chemie International Edition 2021, 60 (29), 15870-15878.

34. Kesimer, M.; Ehre, C.; Burns, K. A.; Davis, C. W.; Sheehan, J. K.; Pickles, R. J. Molecular organization of the mucins and glycocalyx underlying mucus transport over mucosal surfaces of the airways. Mucosal immunology 2013, 6 (2), 379-392.

35. Granata, A.; Argyropoulos, D. S. 2-Chloro-4,4,5,5-tetramethyl-1,3,2-dioxaphospholane, a Reagent for the Accurate Determination of the Uncondensed and Condensed Phenolic Moieties in Lignins. Journal of Agricultural and Food Chemistry 1995, 43 (6), 1538-1544. DOI: 10.1021/jf00054a023.

36. Bhattacharjee, S. DLS and zeta potential - What they are and what they are not? Journal of Controlled Release 2016, 235, 337-351. DOI: https://doi.org/10.1016/j.jconrei.2016.06.017.

37. Lowry, G. V.; Hill, R. J.; Harper, S.; Rawle, A. F.; Hendren, C. O.; Klaessig, F.; Nobbmann, U.; Sayre, P.; Rumble, J. Guidance to improve the scientific value of zeta-potential measurements in nanoEHS. Environmental Science: Nano 2016, 3 (5), 953-965.

38. Kazachenko, A. S.; Akman, F.; Vasilieva, N. Y.; Malyar, Y. N.; Fetisova, O. Y.; Lutoshkin, M. A.; Berezhnaya, Y. D.; Miroshnikova, A. V.; Issaoui, N.; Xiang, Z. Sulfation of Wheat Straw Soda Lignin with Sulfamic Acid over Solid Catalysts. Polymers 2022, 14 (15), 3000.

39. Alam, M. N.; Christopher, L. P. Natural Cellulose-Chitosan Cross-Linked Superabsorbent Hydrogels with Superior Swelling Properties. ACS Sustainable Chemistry & Engineering 2018, 6 (7), 8736-8742. DOI: 10.1021/acssuschemeng.8b01062.

40. Lafforgue, O.; Seyssiecq, I.; Poncet, S.; Favier, J. Rheological properties of synthetic mucus for airway clearance. Journal of Biomedical Materials Research Part A 2018, 106 (2), 386-396.

41. Vinod, A.; Tadmor, R.; Katoshevski, D.; Gutmark, E. J. Gels That Serve as Mucus Simulants: A Review. Gels 2023, 9 (7), 555.

42. Zhao, Z.; Das, S.; Zharnikov, M. Tuning the Properties of Polyethylene glycol) Films and Membranes by the Molecular Weight of the Precursors. ACS Applied Polymer Materials 2022, 4 (1), 645-653. DOI: 10.1021/acsapm.1c01569.

43. Shukla, D.; Spear, P. G. Herpesviruses and heparan sulfate: an intimate relationship in aid of viral entry. The Journal of clinical investigation 2001, 108 (4), 503-510.

44. Brewster, J. D. A simple micro-growth assay for enumerating bacteria. Journal of microbiological methods 2003, 53 (1), 77-86.

45. Maan, H.; Povolotsky, T. L.; Porat, Z.; Itkin, M.; Malitsky, S.; Kolodkin-Gal, I. Imaging flow cytometry reveals a dual role for exopolysaccharides in biofilms: To promote self-adhesion while repelling non-self-community members. Computational and structural biotechnology journal 2022, 20, 15-25.

46. Adeel, M., Farooq, T., White, J. C., Hao, Y., He, Z., & Rui, Y. (2021). Carbon-based nanomaterials suppress tobacco mosaic virus (TMV) infection and induce resistance in Nicotiana benthamiana. Journal of Hazardous Materials, 404, 124167.

47. Chandna, S., Thakur, N. S., Kaur, R., & Bhaumik, J. (2020). Lignin-bimetallic nanoconjugate doped pH-responsive hydrogels for laser-assisted antimicrobial photodynamic therapy. Biomacromolecules, 21(8), 3216-3230.

48. Piqué, N., Miñana-Galbis, D., Merino, S., & Tomás, J. M. (2015). Virulence factors of Erwinia amylovora: a review. *International journal of molecular sciences,* 16(6), 12836-12854.

49. Vonnemann, J., Sieben, C., Wolff, C., Ludwig, K., Böttcher, C., Herrmann, A., & Haag, R. (2014). Virus inhibition induced by polyvalent nanoparticles of different sizes. Nanoscale, 6(4), 2353-2360.

50. J. B. Ristaino, P. K. Anderson, D. P. Bebber, K. A. Brauman, N. J. Cunniffe, N. V. Fedoroff, ... Q.Wei, (2021). The persistent threat of emerging plant disease pandemics to global food security. Proceedings of the National Academy of Sciences, 118(23), e2022239118.

51. T. M. Clausen, D. R. Sandoval, C. B. Spliid, J. Pihl, H. R. Perrett, C. D. Painter, A. Narayanan,.. J. D. Esko.(2020) SARS-CoV-2 Infection Depends on Cellular Heparan Sulfate and ACE2. Cell, 183, 1043-1057.e1015.

52. M. Koehler, M. Delguste, C. Sieben, L. Gillet, D. Alsteens.(2020) Initial Step of Virus Entry: Virion Binding to Cell-Surface Glycans. Annu. Rev. Virol., 7, 143-165.

53. Wallert, M., Nie, C., Anilkumar, P., Abbina, S., Bhatia, S., Ludwig, K., ... Haag, R., Block, S. (2020). Mucin-Inspired, High Molecular Weight Virus Binding Inhibitors Show Biphasic Binding Behavior to Influenza A Viruses. Small, 16(47), 2004635.

54. P. Dey, T. Bergmann, J. L. Cuellar-Camacho, S. Ehrmann, M. S. Chowdhury, M. Zhang, I. Dahmani, R. Haag, W. Azab. (2018) Multivalent Flexible Nanogels Exhibit Broad-Spectrum Antiviral Activity by Blocking Virus Entry. ACS Nano 12, 6429-6442.

55. C. Nie, M. Stadtmüller, B. Parshad, M. Wallert, V. Ahmadi, Y. Kerkhoff, S. Bhatia, S. Block, C. Cheng, T. Wolff, R.

Haag. (2021) Heteromultivalent topology-matched nanostructures as potent and broad-spectrum influenza A virus inhibitors. Sci. Adv. 7, eabd3803.

56. C. Nie, M. Stadtmüller, H. Yang, Y. Xia, T. Wolff, C. Cheng, R. Haag. (2020) Spiky Nanostructures with Geometry-matching Topography for Virus Inhibition. Nano Lett., 20, 5367-5375.

57. S. Donskyi, C. Nie, K. Ludwig, J. Trimpert, R. Ahmed, E. Quaas, K. Achazi, J. Radnik, M. Adeli, R. Haag, K. Osterrieder. (2021) SARS-CoV-2 Inhibitors: Graphene Sheets with Defined Dual Functionalities for the Strong SARS-CoV-2 Interactions (Small 11/2021). Small, 17, 2170046.

58. S. Bhatia, D. Lauster, M. Bardua, K. Ludwig, S. Angioletti-Uberti, N. Popp, U. Hoffmann, F. Paulus, M. Budt, M. Stadtmüller, T. Wolff, A. Hamann, C. Böttcher, A. Herrmann, R. Haag.(2017) Linear polysialoside outperforms dendritic analogs for inhibition of influenza virus infection in vitro and in vivo. Biomaterials, 138, 22-34.

59. M. N. Stadtmueller, S. Bhatia, P. Kiran, M. Hilsch, V. Reiter-Scherer, L. Adam, B. Parshad, M. Budt, S. Klenk, K. Sellrie, D. Lauster, P. H. Seeberger, C. P. R. Hackenberger, A. Herrmann, R. Haag, T. Wolff. (2021) Evaluation of Multivalent Sialylated Polyglycerols for Resistance and Broad Antiviral Activity against Influenza Viruses.

60. A. N. Baker, S.-J. Richards, C. S. Guy, T. R. Congdon, M. Hasan, A. J. Zwetsloot, A. Gallo, J. R. Lewandowski, P. J. Stansfeld, A. Straube, M. Walker, S. Chessa, G. Pergolizzi, S. Dedola, R. A. Field, M. I. Gibson.(2020) The SARS-COV-2 Spike Protein Binds Sialic Acids and Enables Rapid Detection in a Lateral Flow Point of Care Diagnostic Device. ACS Cent. Sci., 6, 2046-2052.

61. Ren, X., Gao, R., van der Mei, H. C., Ren, Y., Peterson, B. W., & Busscher, H. J. (2020). Eradicating infecting bacteria while maintaining tissue integration on photothermal nanoparticle-coated titanium surfaces. ACS Applied Materials & Interfaces, 12(31), 34610-34619.

62. Imani, S. M., Ladouceur, L., Marshall, T., Maclachlan, R., Soleymani, L., & Didar, T. F. (2020). Antimicrobial nanomaterials and coatings: Current mechanisms and future perspectives to control the spread of viruses including SARS-CoV-2. ACS Nano, 14(10), 12341-12369.

63. Vieira, F. R., Magina, S., Evtuguin, D. V., & Barros-Timmons, A. (2022). Lignin as a Renewable Building Block for Sustainable Polyurethanes. Materials, 15(17), 6182.

## Claims

1. Use of sulfated lignin, excluding methods for treatment of the human or animal body by surgery or therapy and diagnostic methods practiced on the human or animal body, as compound for adsorbing at least one of a microorganism, a pathogen, pollen, and spores.

2. Use according to claim 1, **characterized in that** the sulfated lignin is used as solid, in form of a hydrogel, or in form of a spray.

3. Use according to claim 1 or 2, **characterized in that** the sulfated lignin has a degree of sulfation lying in a range from 10 % to 100 %.

4. Use according to any of the preceding claims, **characterized in that** the sulfated lignin is sulfated kraft lignin.

5. Use according to any of the preceding claims, **characterized in that** the sulfated lignin is used for water purification.

6. Use of sulfated lignin, excluding methods for treatment of the human or animal body by surgery or therapy and diagnostic methods practiced on the human or animal body, as water storing element in a water retention system.

7. Sulfated lignin for use as a medicament.

8. Sulfated lignin for use according to claim 7, **characterized in that** the medicament is configured to be applied to a patient's skin.

**9.** Sulfated lignin for use according to claim 7, **characterized in that** the medicament is a mucus replacement.

**10.** Sulfated lignin for use as a drug delivery compound.

**11.** Article comprising sulfated lignin.

**12.** Article according to claim 11, **characterized in that** the article comprises an article core having a surface, wherein the sulfated lignin is present on the surface in form of a coating.

**13.** Article according to claim 11 or 12, **characterized in that** the sulfated lignin forms part of a swellable polymer network that is configured to absorb water or an aqueous solution and to form a hydrogel upon absorbing water or an aqueous solution.

**14.** Article according to any of claims 11 to 13, **characterized in that** the article is a medical, veterinary, or sanitary article.

**15.** Method for preparing a hydrogel comprising sulfated lignin, comprising the following steps:

a) providing an aqueous solution of sulfated lignin;
b) adding an aqueous solution of polyacrylic acid to the aqueous solution of sulfated lignin to obtain a reaction mixture;
c) stirring the reaction mixture for a first period of time;
d) adding a free radical initiator to the reaction mixture;
e) allowing a formation of a lignin acryl ester and a polymerization of the lignin acryl ester with acrylic acid to obtain a hydrogel during a second period of time; and
f) optionally purifying the obtained hydrogel.

FIG 1A

SO₃(Py)x at 60 °C for 20 h

FIG 1B

FIG 2A

EP 4 609 869 A1

FIG 2B

FIG 2C

FIG 3A

FIG 3B

FIG 3C

FIG 3D

FIG 3E

FIG 3F

FIG 3G

FIG 3H

FIG 4A

FIG 4B

FIG 5A

FIG 5B

**EUROPEAN SEARCH REPORT**

**Application Number**

EP 24 16 0979

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 01/35747 A2 (PLANT RES INT BV [NL]; KRIEKEN WILHELMUS MARIA V D [NL] ET AL.) 25 May 2001 (2001-05-25) | 1,2,11 | INV.<br>A61K31/737<br>A61K47/36 |
| Y | * page 1, paragraph 1-7 *<br>* page 4, lines 27-38 *<br>* page 5, line 1 - page 6, line 13 *<br>* example 1 * | 1-5,<br>11-13 | A61P17/00<br>A61P31/00 |
| X | CN 113 912 192 A (YANTAI INST COASTAL ZONE RES CAS) 11 January 2022 (2022-01-11) | 1,2,5,<br>11,13 | |
| Y | * example 5 *<br>* claims 1-4 *<br>* The passages of the enclosed automatic translation referred to in the written opinion. * | 1-5,<br>11-13 | |
| A | CN 114 989 830 A (JIANGSU QINGKE MUCHENG NEW MATERIAL RES INSTITUTE CO LTD) 2 September 2022 (2022-09-02)<br>* table 1 *<br>* The passages of the enclosed automatic translation referred to in the written opinion. * | 1-5,<br>11-13 | |
| | | | TECHNICAL FIELDS SEARCHED (IPC) |
| | | | A61K<br>C02F |
| Y | OEYEN MEREL ET AL: "A unique class of lignin derivatives displays broad anti-HIV activity by interacting with the viral envelope",<br>VIRUS RESEARCH, AMSTERDAM, NL,<br>vol. 274, 13 October 2019 (2019-10-13),<br>XP085882745,<br>ISSN: 0168-1702, DOI:<br>10.1016/J.VIRUSRES.2019.197760<br>[retrieved on 2019-10-13]<br>* abstract *<br>* figure 2; tables 1-2, 4 *<br>* page 6, right-hand column * | 1-5,<br>11-13 | A61L<br>A61P |

-/--

The present search report has been drawn up for all claims

| Place of search | Date of completion of the search | Examiner |
|---|---|---|
| The Hague | 1 August 2024 | Olausson Boulois, J |

EPO FORM 1503 03.82 (P04C01)

Europäisches
Patentamt

European
Patent Office

Office européen
des brevets

**EUROPEAN SEARCH REPORT**

**Application Number**

EP 24 16 0979

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | YU CHENGHUA ET AL: "The synthesis and absorption dynamics of a lignin-based hydrogel for remediation of cationic dye-contaminated effluent", REACTIVE AND FUNCTIONAL POLYMERS, ELSEVIER, AMSTERDAM, NL, vol. 106, 22 July 2016 (2016-07-22), pages 137-142, XP029687801, ISSN: 1381-5148, DOI: 10.1016/J.REACTFUNCTPOLYM.2016.07.016 * abstract * * pages 137-138 * * figure 1 * | 1-5, 11-13 | |
| Y | PEREIRA ANDERSON DO ESPIRITO SANTO ET AL: "Lignin nanoparticles: New insights for a sustainable agriculture", JOURNAL OF CLEANER PRODUCTION, ELSEVIER, AMSTERDAM, NL, vol. 345, 28 February 2022 (2022-02-28), XP087003147, ISSN: 0959-6526, DOI: 10.1016/J.JCLEPRO.2022.131145 [retrieved on 2022-02-28] * abstract * * figures 5-7 * | 1-5, 11-13 | TECHNICAL FIELDS SEARCHED (IPC) |

The present search report has been drawn up for all claims

| Place of search | Date of completion of the search | Examiner |
|---|---|---|
| The Hague | 1 August 2024 | Olausson Boulois, J |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

 .................................................................................

& : member of the same patent family, corresponding
    document

EPO FORM 1503 03.82 (P04C01)

Europäisches
Patentamt

European
Patent Office

Office européen
des brevets

Application Number

EP 24 16 0979

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

    see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

    1-5(completely); 11-13(partially)

☐ The present supplementary European search report has been drawn up for those parts of the European patent application which relate to the invention first mentioned in the claims (Rule 164 (1) EPC).

34

Europäisches
Patentamt

European
Patent Office

Office européen
des brevets

**LACK OF UNITY OF INVENTION
SHEET B**

Application Number

EP 24 16 0979

The Search Division considers that the present European patent application does not comply with the requirements of unity of invention and relates to several inventions or groups of inventions, namely:

1. claims: 1-5(completely); 11-13(partially)

     Use of sulfated lignin, excluding methods for treatment of the human or animal body by surgery or therapy and diagnostic methods practiced on the human or animal body, as compound for adsorbing at least one of a microorganism, a pathogen, pollen, and spores. Article comprising sulfated lignin suitable for such a use.
     - - -

2. claims: 6(completely); 11-13(partially)

     Use of sulfated lignin, excluding methods for treatment of the human or animal body by surgery or therapy and diagnostic methods practiced on the human or animal body, as water storing element in a water retention system. Article comprising sulfated lignin suitable for such a use.
     - - -

3. claims: 7-9(completely); 11-14(partially)

     Sulfated lignin for use as a medicament. Article comprising sulfated lignin suitable for such a use.
     - - -

4. claims: 10(completely); 11-14(partially)

     Sulfated lignin for use as a drug delivery compound. Article comprising sulfated lignin suitable for such a use.
     - - -

5. claim: 15

     Method for preparing a hydrogel comprising sulfated lignin, comprising the following steps: a) providing an aqueous solution of sulfated lignin ;b) adding an aqueous solution of polyacrylic acid to the aqueous solution of sulfated lignin to obtain a reaction mixture; c) stirring the reaction mixture for a first period of time;d) adding a free radical initiator to the reaction mixture; e) allowing a formation of a lignin acryl ester and a polymerization of the lignin acryl ester with acrylic acid to obtain a hydrogel during a second period of time; and f) optionally purifying the obtained hydrogel.
     - - -

## ANNEX TO THE EUROPEAN SEARCH REPORT
## ON EUROPEAN PATENT APPLICATION NO.

EP 24 16 0979

This annex lists the patent family members relating to the patent documents cited in  the above-mentioned European search report.
The members are as contained in the European Patent Office EDP file on
The European Patent Office is in no way liable for these particulars which are merely given for the purpose of information.

01-08-2024

| Patent document cited in search report | | Publication date | Patent family member(s) | | Publication date |
|---|---|---|---|---|---|
| WO 0135747 | A2 | 25-05-2001 | AU | 2836601 A | 30-05-2001 |
| | | | EP | 1229794 A2 | 14-08-2002 |
| | | | WO | 0135747 A2 | 25-05-2001 |
| CN 113912192 | A | 11-01-2022 | NONE | | |
| CN 114989830 | A | 02-09-2022 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2688611 A
- US 5013825 A
- US 5049661 A
- US 5043434 A

**Non-patent literature cited in the description**

- **BEAUCAMP, A.** ; **MUDDASAR, M.** ; **AMIINU, I. S.** ; **LEITE, M. M.** ; **CULEBRAS, M.** ; **LATHA, K.** ; **GUTIÉRREZ, M. C.** ; **RODRIGUEZ-PADRON, D.** ; **DEL MONTE, F.** ; **KENNEDY, T.** Lignin for energy applications-state of the art, life cycle, technoeconomic analysis and future trends. *Green Chemistry*, 2022
- **JI, D.** ; **PARK, J. M.** ; **OH, M. S.** ; **NGUYEN, T. L.** ; **SHIN, H.** ; **KIM, J. S.;** ; **KIM, D.;** ; **PARK, H. S.** ; **KIM, J.** Superstrong, superstiff, and conductive alginate hydrogels. *Nature Communications*, 2022, vol. 13 (1), 3019
- **BEJ, R.** ; **HAAG, R.** Mucus-Inspired Dynamic Hydrogels: Synthesis and Future Perspectives. *Journal of the American Chemical Society*, 2022, vol. 144 (44), 20137-20152
- **LEAL, J.** ; **SMYTH, H. D. C.** ; **GHOSH, D.** Physicochemical properties of mucus and their impact on transmucosal drug delivery. *Int J Pharm*, 2017, vol. 532 (1), 555-572
- **MCGUCKIN, M. A.** ; **LINDÉN, S. K.** ; **SUTTON, P** ; **FLORIN, T. H.** Mucin dynamics and enteric pathogens. *Nature Reviews Microbiology*, 2011, vol. 9 (4), 265-278
- **YANG, S.** ; **DUNCAN, G. A.** Synthetic mucus biomaterials for antimicrobial peptide delivery. *Journal of Biomedical Materials Research Part A*, 2023
- **ZANIN, M.** ; **BAVISKAR, P.** ; **WEBSTER, R.** ; **WEBBY, R.** The Interaction between Respiratory Pathogens and Mucus. *Cell Host & Microbe*, 2016, vol. 19 (2), 159-168
- **LIELEG, O.** ; **LIELEG, C.** ; **BLOOM, J.** ; **BUCK, C. B.** ; **RIBBECK, K.** Mucin Biopolymers As Broad-Spectrum Antiviral Agents. *Biomacromolecules*, 2012, vol. 13 (6), 1724-1732
- **SHARMA, A.** ; **THONGROM, B.** ; **BHATIA, S.** ; **VON LOSPICHL, B.** ; **ADDANTE, A.** ; **GRAEBER, S. Y.** ; **LAUSTER, D.** ; **MALL, M. A.** ; **GRADZIELSKI, M.** ; **HAAG, R.** Polyglycerol-Based Mucus-Inspired Hydrogels. *Macromolecular Rapid Communications*, 2021, vol. 42 (20), 2100303
- **YAN, H.** ; **MELIN, M.** ; **JIANG, K.** ; **TROSSBACH, M** ; **BADADAMATH, B.** ; **LANGER, K.** ; **WINKELJANN, B.** ; **LIELEG, O.** ; **HONG, J.** ; **JOENSSON, H. N**. Immune-Modulating Mucin Hydrogel Microdroplets for the Encapsulation of Cell and Microtissue. *Advanced Functional Materials*, 2021, vol. 31 (42), 2105967
- **ABDEL AZIZ, M. H** ; **MOSIER, P. D** ; **DESAI, U. R.** Identification of the site of binding of sulfated, low molecular weight lignins on thrombin. *Biochemical and Biophysical Research Communications*, 2011, vol. 413 (2), 348-352, https://doi.org/10.1016/j.bbrc.2011.08.102
- **AFEWERKI, S.** ; **WANG, X.** ; **RUIZ-ESPARZA, G. U.** ; **TAI, C.-W.** ; **KONG, X.** ; **ZHOU, S** ; **WELCH, K.** ; **HUANG, P** ; **BENGTSSON, R.** ; **XU, C. et al.** Combined Catalysis for Engineering Bioinspired, Lignin-Based, Long-Lasting, Adhesive, Self-Mending, Antimicrobial Hydrogels. *ACS Nano*, 2020, vol. 14 (12), 17004-17017
- **MENNANI, M.** ; **KASBAJI, M.** ; **BENHAMOU, A. A.** ; **BOUSSETTA, A.** ; **MEKKAOUI, A. A.** ; **GRIMI, N** ; **MOUBARIK, A.** Current approaches, emerging developments and functional prospects for lignin-based catalysts-Review. *Green Chemistry*, 2023
- **WAN, Y.** ; **HE, J.** ; **ZHANG, Y** ; **CHEN, E. Y. X.** One-Step Synthesis of Lignin-Based Triblock Copolymers as High-Temperature and UV-Blocking Thermoplastic Elastomers. *Angewandte Chemie*, 2022, vol. 134 (8), e202114946
- **MALYAR, Y. N.** ; **KAZACHENKO, A. S.** ; **VASILYEVA, N. Y.** ; **FETISOVA, O. Y.** ; **BOROVKOVA, V. S.** ; **MIROSHNIKOVA, A. V.;** ; **LEVDANSKY, A. V.;** ; **SKRIPNIKOV, A. M.** Sulfation of wheat straw soda lignin: Role of solvents and catalysts. *Catalysis Today*, 2022, 397-399, 397-406, https://doi.org/10.1016/j.cattod.2021.07.033

- **LEVDANSKY, A. V.** ; **VASILYEVA, N. Y.** ; **MALYAR, Y. N.** ; **KONDRASENKO, A. A.** ; **FETISOVA, O. Y.** ; **KAZACHENKO, A. S.** ; **LEVDANSKY, V. A.** ; **KUZNETSOV, B. N. A**. n Efficient Method of Birch Ethanol Lignin Sulfation with a Sulfaic Acid-Urea Mixture. *Molecules*, 2022, vol. 27 (19), 6356
- **ZHANG, C.** ; **SHEN, X.** ; **JIN, Y.** ; **CHENG, J.** ; **CAI, C.** ; **WANG, F.** Catalytic strategies and mechanism analysis orbiting the center of critical intermediates in lignin depolymerization. *Chemical Reviews*, 2023, vol. 123 (8), 4510-4601
- **SHARMA, V.** ; **TSAI, M.-L.** ; **NARGOTRA, P.** ; **CHEN, C.-W.** ; **SUN, P.-P.** ; **SINGHANIA, R. R** ; **PATEL, A. K.** ; **DONG, C.-D.** Journey of lignin from a roadblock to bridge for lignocellulose biorefineries: A comprehensive review. *Science of The Total Environment*, 2023, vol. 861, 160560
- **SETHUPATHY, S.** ; **MORALES, G. M** ; **GAO, L.** ; **WANG, H.** ; **YANG, B.** ; **JIANG, J.** ; **SUN, J.** ; **ZHU, D.** Lignin valorization: Status, challenges and opportunities. *Bioresource Technology*, 2022, vol. 347, 126696
- **LIU, M.** ; **HAN, B.** ; **DYSON, P. J.** Simultaneous generation of methyl esters and CO in lignin transformation. *Angewandte Chemie International Edition*, 2022, vol. 61 (40), e202209093
- **AGUSTIANY, E. A.** ; **RASYIDUR RIDHO, M.** ; **RAHMI DN, M.** ; **MADYARATRI, E. W.** ; **FALAH, F.** ; **LUBIS, M. A. R.** ; **SOLIHAT, N. N.** ; **SYAMANI, F. A.** ; **KARUNGAMYE, P.** ; **SOHAIL, A.** Recent developments in lignin modification and its application in lignin-based green composites: A review. *Polymer Composites*, 2022, vol. 43 (8), 4848-4865
- **SANTO PEREIRA, A. D. E.** ; **DE OLIVEIRA, J. L.** ; **SAVASSA, S. M.** ; **ROGÉRIO, C. B.** ; **DE MEDEIROS, G. A.** ; **FRACETO, L. F.** Lignin nanoparticles: New insights for a sustainable agriculture. *Journal of Cleaner Production*, 2022, vol. 345, 131145
- **HATAKEYAMA, H.** ; **HATAKEYAMA, T.** Lignin Structure, Properties, and Applications. *Advances in Polymer Science*, 2009, vol. 232, 1-63
- **LIU, X.** ; **INDA, M. E.** ; **LAI, Y.** ; **LU, T. K.** ; **ZHAO, X.** Engineered living hydrogels. *Advanced Materials*, 2022, vol. 34 (26), 2201326
- **YANG, D.** Recent advances in hydrogels. *ACS Publications*, 2022, vol. 34, 1987-1989
- **BLACHE, U.** ; **FORD, E. M.** ; **HA, B.** ; **RIJNS, L.** ; **CHAUDHURI, O.** ; **DANKERS, P. Y.** ; **KLOXIN, A. M.** ; **SNEDEKER, J. G.** ; **GENTLEMAN, E.** Engineered hydrogels for mechanobiology. *Nature Reviews Methods Primers*, 2022, vol. 2 (1), 98
- **JOONAKI, E.** ; **HASSANPOURYOUZBAND, A.** ; **HELDT, C. L.** ; **AREO, O.** Surface chemistry can unlock drivers of surface stability of SARS-CoV-2 in a variety of environmental conditions. *Chem*, 2020, vol. 6 (9), 2135-2146
- **CAMPBELL, K. T** ; **WYSOCZYNSKI, K.** ; **HADLEY, D. J.** ; **SILVA, E. A.** Computational-based design of hydrogels with predictable mesh properties. *ACS biomaterials science & engineering*, 2019, vol. 6 (1), 308-319
- **CONNOLLY, S. A.** ; **JARDETZKY, T. S.** ; **LONG-NECKER, R.** The structural basis of herpesvirus entry. *Nature reviews Microbiology*, 2021, vol. 19 (2), 110-121
- **POUYAN, P.** ; **NIE, C.** ; **BHATIA, S.** ; **WEDEPOHL, S.** ; **ACHAZI, K** ; **OSTERRIEDER, N.** ; **HAAG, R.** Inhibition of Herpes Simplex Virus Type 1 Attachment and Infection by Sulfated Polyglycerols with Different Architectures. *Biomacromolecules*, 2021, vol. 22 (4), 1545-1554
- **THONGROM, B.** ; **SHARMA, A.** ; **NIE, C.** ; **QUAAS, E.** ; **RAUE, M.** ; **BHATIA, S.** ; **HAAG, R.** Scaffold Flexibility Controls Binding of Herpes Simplex Virus Type 1 with Sulfated Dendritic Polyglycerol Hydrogels Fabricated by Thiol-Maleimide Click Reaction. *Macromolecular Bioscience*, 2022, vol. 22 (5), 2100507
- **AHMADI, V.** ; **NIE, C.** ; **MOHAMMADIFAR, E.** ; **ACHAZI, K.** ; **WEDEPOHL, S.** ; **KERKHOFF, Y.** ; **BLOCK, S.;** ; **OSTERRIEDER, K.;** ; **HAAG, R.** One-pot gram-scale synthesis of virucidal heparin-mimicking polymers as HSV-1 inhibitors. *Chemical Communications*, 2021, vol. 57 (90), 11948-11951
- **NIE, C.** ; **POUYAN, P.** ; **LAUSTER, D** ; **TRIMPERT, J.** ; **KERKHOFF, Y.** ; **SZEKERES, G. P.** ; **WALLERT, M.** ; **BLOCK, S.** ; **SAHOO, A. K.** ; **DERNEDDE, J.** Polysulfates Block SARS-CoV-2 Uptake through Electrostatic Interactions. *Angewandte Chemie International Edition*, 2021, vol. 60 (29), 15870-15878
- **KESIMER, M.** ; **EHRE, C.** ; **BURNS, K. A.** ; **DAVIS, C. W.** ; **SHEEHAN, J. K.** ; **PICKLES, R. J.** Molecular organization of the mucins and glycocalyx underlying mucus transport over mucosal surfaces of the airways. *Mucosal immunology*, 2013, vol. 6 (2), 379-392
- **GRANATA, A** ; **ARGYROPOULOS, D. S.** 2-Chloro-4,4,5,5-tetramethyl-1,3,2-dioxaphospholane, a Reagent for the Accurate Determination of the Uncondensed and Condensed Phenolic Moieties in Lignins. *Journal of Agricultural and Food Chemistry*, 1995, vol. 43 (6), 1538-1544
- **BHATTACHARJEE, S.** DLS and zeta potential - What they are and what they are not?. *Journal of Controlled Release*, 2016, vol. 235, 337-351
- **LOWRY, G. V.** ; **HILL, R. J.** ; **HARPER, S.** ; **RAWLE, A. F.** ; **HENDREN, C. O.** ; **KLAESSIG, F.** ; **NOBBMANN, U.** ; **SAYRE, P.** ; **RUMBLE, J.** Guidance to improve the scientific value of zeta-potential measurements in nanoEHS. *Environmental Science: Nano*, 2016, vol. 3 (5), 953-965

- **KAZACHENKO, A. S.** ; **AKMAN, F.** ; **VASILIEVA, N. Y.** ; **MALYAR, Y. N.** ; **FETISOVA, O. Y.** ; **LUTOSHKIN, M. A.** ; **BEREZHNAYA, Y. D.** ; **MIROSHNIKOVA, A. V.** ; **ISSAOUI, N.** ; **XIANG, Z.** Sulfation of Wheat Straw Soda Lignin with Sulfamic Acid over Solid Catalysts. *Polymers*, 2022, vol. 14 (15), 3000
- **ALAM, M. N.** ; **CHRISTOPHER, L. P.** Natural Cellulose-Chitosan Cross-Linked Superabsorbent Hydrogels with Superior Swelling Properties. *ACS Sustainable Chemistry & Engineering*, 2018, vol. 6 (7), 8736-8742
- **LAFFORGUE, O.** ; **SEYSSIECQ, I.** ; **PONCET, S.** ; **FAVIER, J.** Rheological properties of synthetic mucus for airway clearance. *Journal of Biomedical Materials Research Part A*, 2018, vol. 106 (2), 386-396
- **VINOD, A.** ; **TADMOR, R.** ; **KATOSHEVSKI, D.** ; **GUTMARK, E. J.** Gels That Serve as Mucus Simulants: A Review. *Gels*, 2023, vol. 9 (7), 555
- **ZHAO, Z.** ; **DAS, S.** ; **ZHARNIKOV, M.** Tuning the Properties of Polyethylene glycol) Films and Membranes by the Molecular Weight of the Precursors. *ACS Applied Polymer Materials*, 2022, vol. 4 (1), 645-653
- **SHUKLA, D.** ; **SPEAR, P. G.** Herpesviruses and heparan sulfate: an intimate relationship in aid of viral entry. *The Journal of clinical investigation*, 2001, vol. 108 (4), 503-510
- **BREWSTER, J. D.** A simple micro-growth assay for enumerating bacteria. *Journal of microbiological methods*, 2003, vol. 53 (1), 77-86
- **MAAN, H.** ; **POVOLOTSKY, T. L.** ; **PORAT, Z.** ; **ITKIN, M** ; **MALITSKY, S.** ; **KOLODKIN-GAL, I.** Imaging flow cytometry reveals a dual role for exopolysaccharides in biofilms: To promote self-adhesion while repelling non-self-community members. *Computational and structural biotechnology journal*, 2022, vol. 20, 15-25
- **ADEEL, M.** ; **FAROOQ, T** ; **WHITE, J. C.** ; **HAO, Y.** ; **HE, Z.** ; **RUI, Y.** Carbon-based nanomaterials suppress tobacco mosaic virus (TMV) infection and induce resistance in Nicotiana benthamiana. *Journal of Hazardous Materials*, 2021, vol. 404, 124167
- **CHANDNA, S.** ; **THAKUR, N. S.** ; **KAUR, R.** ; **BHAUMIK, J.** Lignin-bimetallic nanoconjugate doped pH-responsive hydrogels for laser-assisted antimicrobial photodynamic therapy. *Biomacromolecules*, 2020, vol. 21 (8), 3216-3230
- **VONNEMANN, J.** ; **SIEBEN, C.** ; **WOLFF, C.** ; **LUDWIG, K.** ; **BÖTTCHER, C.** ; **HERRMANN, A.** ; **HAAG, R.** Virus inhibition induced by polyvalent nanoparticles of different sizes. *Nanoscale*, 2014, vol. 6 (4), 2353-2360
- **J. B. RISTAINO** ; **P. K. ANDERSON** ; **D. P. BEBBER** ; **K. A. BRAUMAN** ; **N. J. CUNNIFFE** ; **N. V. FEDOROFF** ; **Q.WEI**. The persistent threat of emerging plant disease pandemics to global food security. *Proceedings of the National Academy of Sciences*, 2021, vol. 118 (23), e2022239118
- **T. M. CLAUSEN** ; **D. R. SANDOVAL** ; **C. B. SPLIID** ; **J. PIHL** ; **H. R. PERRETT** ; **C. D. PAINTER** ; **A. NARAYANAN** ; **J. D. ESKO**. SARS-CoV-2 Infection Depends on Cellular Heparan Sulfate and ACE2. *Cell*, 2020, vol. 183, 1043-1057, e1015
- **M. KOEHLER** ; **M. DELGUSTE** ; **C. SIEBEN** ; **L. GILLET** ; **D. ALSTEENS**. Initial Step of Virus Entry: Virion Binding to Cell-Surface Glycans. *Annu. Rev. Virol.*, 2020, vol. 7, 143-165
- **WALLERT, M.** ; **NIE, C.** ; **ANILKUMAR, P.** ; **ABBINA, S.** ; **BHATIA, S.** ; **LUDWIG, K** ; **HAAG, R.** ; **BLOCK, S.** Mucin-Inspired, High Molecular Weight Virus Binding Inhibitors Show Biphasic Binding Behavior to Influenza A Viruses. *Small*, 2020, vol. 16 (47), 2004635
- **P. DEY** ; **T. BERGMANN** ; **J. L. CUELLAR-CAMACHO** ; **S. EHRMANN** ; **M. S. CHOWDHURY** ; **M. ZHANG** ; **I. DAHMANI** ; **R. HAAG** ; **W. AZAB**. Multivalent Flexible Nanogels Exhibit Broad-Spectrum Antiviral Activity by Blocking Virus Entry. *ACS Nano*, 2018, vol. 12, 6429-6442
- **C. NIE** ; **M. STADTMÜLLER** ; **B. PARSHAD** ; **M. WALLERT** ; **V. AHMADI** ; **Y. KERKHOFF** ; **S. BHATIA** ; **S. BLOCK** ; **C. CHENG** ; **T. WOLFF**. Heteromultivalent topology-matched nanostructures as potent and broad-spectrum influenza A virus inhibitors. *Sci. Adv.*, 2021, vol. 7, 3803
- **C. NIE** ; **M. STADTMÜLLER** ; **H. YANG** ; **Y. XIA** ; **T. WOLFF** ; **C. CHENG** ; **R. HAAG**. Spiky Nanostructures with Geometry-matching Topography for Virus Inhibition. *Nano Lett.*, 2020, vol. 20, 5367-5375
- **S. DONSKYI** ; **C. NIE** ; **K. LUDWIG** ; **J. TRIMPERT** ; **R. AHMED** ; **E. QUAAS** ; **K. ACHAZI** ; **J. RADNIK** ; **M. ADELI** ; **R. HAAG**. SARS-CoV-2 Inhibitors: Graphene Sheets with Defined Dual Functionalities for the Strong SARS-CoV-2 Interactions (Small 11/2021). *Small*, 2021, vol. 17, 2170046
- **S. BHATIA** ; **D. LAUSTER** ; **M. BARDUA** ; **K. LUDWIG** ; **S. ANGIOLETTI-UBERTI** ; **N. POPP** ; **U. HOFFMANN** ; **F. PAULUS** ; **M. BUDT** ; **M. STADTMÜLLER**. Linear polysialoside outperforms dendritic analogs for inhibition of influenza virus infection in vitro and in vivo. *Biomaterials*, 2017, vol. 138, 22-34
- **M. N. STADTMUELLER** ; **S. BHATIA** ; **P. KIRAN** ; **M. HILSCH** ; **V. REITER-SCHERER** ; **L. ADAM** ; **B. PARSHAD** ; **M. BUDT** ; **S. KLENK** ; **K. SELLRIE**. *Evaluation of Multivalent Sialylated Polyglycerols for Resistance and Broad Antiviral Activity against Influenza Viruses*, 2021

- **A. N. BAKER** ; **S.-J. RICHARDS** ; **C. S. GUY** ; **T. R. CONGDON** ; **M. HASAN** ; **A. J. ZWETSLOOT** ; **A. GALLO** ; **J. R. LEWANDOWSKI** ; **P. J. STANSFELD** ; **A. STRAUBE**. The SARS-COV-2 Spike Protein Binds Sialic Acids and Enables Rapid Detection in a Lateral Flow Point of Care Diagnostic Device. *ACS Cent. Sci.*, 2020, vol. 6, 2046-2052

- **REN, X.** ; **GAO, R.** ; **VAN DER MEI, H. C.** ; **REN, Y.** ; **PETERSON, B. W.** ; **BUSSCHER, H. J.** Eradicating infecting bacteria while maintaining tissue integration on photothermal nanoparticle-coated titanium surfaces. *ACS Applied Materials & Interfaces*, 2020, vol. 12 (31), 34610-34619

- **IMANI, S. M.** ; **LADOUCEUR, L.** ; **MARSHALL, T.** ; **MACLACHLAN, R** ; **SOLEYMANI, L.** ; **DIDAR, T. F.** Antimicrobial nanomaterials and coatings: Current mechanisms and future perspectives to control the spread of viruses including SARS-CoV-2. *ACS Nano*, 2020, vol. 14 (10), 12341-12369

- **VIEIRA, F. R.** ; **MAGINA, S** ; **EVTUGUIN, D. V.** ; **BARROS-TIMMONS, A.** Lignin as a Renewable Building Block for Sustainable Polyurethanes. *Materials*, 2022, vol. 15 (17), 6182